(19) Europäisches Patentamt
European Patent Office
Office européen des brevets

(11) EP 3 290 411 B1

(12) # EUROPÄISCHE PATENTSCHRIFT

(45) Veröffentlichungstag und Bekanntmachung des Hinweises auf die Patenterteilung:
**27.11.2019 Patentblatt 2019/48**

(51) Int Cl.:
***C07D 401/10*** (2006.01) ***C09K 11/06*** (2006.01)

(21) Anmeldenummer: **17184257.8**

(22) Anmeldetag: **01.08.2017**

(54) **ORGANISCHE MOLEKÜLE, INSBESONDERE ZUR VERWENDUNG IN ORGANISCHEN OPTOELEKTRONISCHEN VORRICHTUNGEN**

ORGANIC MOLECULES, PARTICULARLY FOR USE IN ORGANIC OPTOELECTRIC DEVICES

MOLECULES ORGANIQUES, EN PARTICULIER POUR APPAREILS ORGANIQUES OPTOELECTRIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.08.2016 DE 102016115997**

(43) Veröffentlichungstag der Anmeldung:
**07.03.2018 Patentblatt 2018/10**

(73) Patentinhaber: **CYNORA GMBH**
**76646 Bruchsal (DE)**

(72) Erfinder: **ZINK, Daniel**
**76646 Bruchsal (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**WO-A1-2013/100540** **WO-A1-2014/146752**
**WO-A1-2016/024675** **KR-A- 20140 139 307**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die Erfindung betrifft rein organische Moleküle und deren Verwendung in organischen lichtemittierenden Dioden (OLEDs) und in anderen organischen optoelektronischen Vorrichtungen.

**Beschreibung**

Der vorliegenden Erfindung lag die Aufgabe zu Grunde, Moleküle bereitzustellen, die sich zur Verwendung in optoelektronischen Vorrichtungen eignen.

KR 20140139307 A beschreibt Verbindungen sowie organische lichtemittierende Dioden enthaltend derartige Verbindungen. Die WO 2014/146752 A1 offenbart Materialien für optoelektronische Vorrichtungen. Aus der WO 2016/024675 A1 sind organische photoelektronische Vorrichtungen und Bildschirmvorrichtungen bekannt. Die WO 2013/100540 A1 beschreibt Verbindungen für organische optoelektronische Vorrichtungen.

Mit der Erfindung wird eine neue Klasse von organischen Molekülen bereitgestellt, die sich zur Verwendung in organischen optoelektronischen Vorrichtungen eignen.

Die erfindungsgemäßen organischen Moleküle sind rein organische Moleküle, weisen also keine Metallionen auf und grenzen sich so von den zur Verwendung in organischen optoelektronischen Vorrichtungen bekannten Metallkomplexverbindungen ab.

Die erfindungsgemäßen organischen Moleküle zeichnen sich durch Emissionen im blauen, himmelblauen oder grünen Spektralbereich aus. Die Photolumineszenzquantenausbeuten der erfindungsgemäßen organischen Moleküle betragen insbesondere 20 % und mehr. Die erfindungsgemäßen Moleküle zeigen insbesondere thermisch aktivierte verzögerte Fluoreszenz (TADF). Die Verwendung der erfindungsgemäßen Moleküle in einer optoelektronischen Vorrichtung, beispielsweise einer organischen lichtemittierenden Diode (OLED), führt zu höheren Effizienzen der Vorrichtung. Entsprechende OLEDs weisen eine höhere Stabilität auf als OLEDs mit bekannten Emittermaterialien und vergleichbarer Farbe.

Unter dem blauen Spektralbereich wird hier der sichtbare Bereich von 430 nm bis 470 nm verstanden. Unter dem himmelblauen Spektralbereich wird hier der Bereich von 470 nm bis 499 nm verstanden. Unter dem grünen Spektralbereich wird hier der Bereich von 500 nm bis 599 nm verstanden. Dabei liegt das Emissionsmaximum im jeweiligen Bereich.

Die organischen Moleküle enthalten:

- eine erste chemische Einheit bestehend aus einer Struktur gemäß Formel I

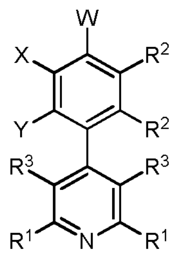

Formel I

und

- eine zweite chemische Einheit D bestehend aus einer Struktur gemäß Formel IIa,

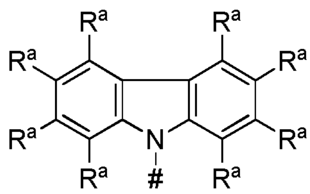

Formel IIa

Hierbei ist die erste chemische Einheit über eine Einfachbindung mit der zweiten chemischen Einheit D verknüpft.

X ist H oder Anknüpfungspunkt der Einfachbindung zwischen der chemischen Einheit gemäß Formel I und der chemischen Einheit D.

Y ist H oder Anknüpfungspunkt der Einfachbindung zwischen der chemischen Einheit gemäß Formel I und der chemischen Einheit D.

W ist CN oder $CF_3$.

# ist Anknüpfungspunkt der Einfachbindung zwischen der chemischen Einheit D und der chemischen Einheit gemäß Formel I.

$R^1$, $R^2$, und $R^3$ ist bei jedem Auftreten gleich oder verschieden H, Deuterium, eine lineare Alkylgruppe mit 1 bis 5 C-Atomen, eine lineare Alkenyl- oder Alkinylgruppe mit 2 bis 8 C-Atomen, eine verzweigte oder cyclische Alkyl-, Alkenyl- oder Alkinylgruppe mit 3 bis 10 C-Atomen, wobei ein oder mehrere H-Atome durch Deuterium ersetzt sein können, oder ein aromatisches oder heteroaromatisches Ringsystem mit 5 bis 15 aromatischen Ringatomen, das jeweils mit einem oder mehreren Resten $R^7$ substituiert sein kann.

$R^a$, $R^4$ und $R^5$ ist bei jedem Auftreten gleich oder verschieden H, Deuterium, $N(R^6)_2$, OH, $Si(R^6)_3$, $B(OR^6)_2$, $OSO_2R^6$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils mit einem oder mehreren Resten $R^6$ substituiert sein kann, oder eine Aryloxy- oder Heteroaryloxygruppe mit 5 bis 60 aromatischen Ringatomen, die mit einem oder mehreren Resten $R^6$ substituiert sein kann, oder eine Diarylaminogruppe, Diheteroarylaminogruppe oder Arylheteroarylaminogruppe mit 10 bis 40 aromatischen Ringatomen, welche mit einem oder mehreren Resten $R^6$ substituiert sein kann.

$R^6$ ist bei jedem Auftreten gleich oder verschieden H, Deuterium, $N(R^7)_2$, OH, $Si(R^7)_3$, $B(OR^7)_2$, $OSO_2R^7$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^7$ substituiert sein kann, wobei eine oder mehrere nicht benachbarte $CH_2$-Gruppen durch $R^7C=CR^7$, C≡C, $Si(R^7)_2$, $Ge(R^7)_2$, $Sn(R^7)_2$, C=O, C=S, C=Se, $C=NR^7$, $P(=O)(R^7)$, SO, $SO_2$, $NR^7$, O, S oder $CONR^7$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils mit einem oder mehreren Resten $R^7$ substituiert sein kann, oder eine Aryloxy- oder Heteroaryloxygruppe mit 5 bis 60 aromatischen Ringatomen, die mit einem oder mehreren Resten $R^7$ substituiert sein kann, oder eine Diarylaminogruppe, Diheteroarylaminogruppe oder Arylheteroarylaminogruppe mit 10 bis 40 aromatischen Ringatomen, welche mit einem oder mehreren Resten $R^7$ substituiert sein kann.

$R^7$ ist bei jedem Auftreten gleich oder verschieden H, Deuterium, OH, $CF_3$, CN, F, eine lineare Alkyl-, Alkoxy- oder Thioalkoxygruppe mit 1 bis 5 C-Atomen oder eine lineare Alkenyl- oder Alkinylgruppe mit 2 bis 5 C-Atomen oder eine verzweigte oder cyclische Alkyl-, Alkenyl-, Alkinyl-, Alkoxy- oder Thioalkoxygruppe mit 3 bis 5 C-Atomen, wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen oder eine Aryloxy- oder Heteroaryloxygruppe mit 5 bis 60 aromatischen Ringatomen oder eine Diarylaminogruppe, Diheteroarylaminogruppe oder Arylheteroarylaminogruppe mit 10 bis 40 aromatischen Ringatomen.

Erfindungsgemäß kann jeder der Reste $R^a$, $R^4$, $R^5$ oder $R^6$ auch mit einem oder mehreren weiteren Resten $R^a$, $R^4$, $R^5$ oder $R^6$ ein mono- oder polycyclisches, aliphatisches, aromatisches und/oder benzoanelliertes Ringsystem bilden.

Erfindungsgemäß ist genau ein Rest ausgewählt aus X und Y gleich dem Anknüpfungspunkt einer Einfachbindung zwischen der chemischen Einheit gemäß Formel I und der chemischen Einheit D.

In einer Ausführungsform ist $R^1$, $R^2$ und $R^3$ bei jedem Auftreten gleich oder verschieden H, Methyl oder Phenyl.

In einer Ausführungsform ist W gleich CN.

In einer Ausführungsform ist $R^a$ bei jedem Auftreten gleich oder verschieden H, Deuterium, $N(R^6)_2$, OH, $Si(R^6)_3$, $B(OR^6)_2$, $OSO_2R^6$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können; oder eine Aryloxy- oder Heteroaryloxygruppe mit 5 bis 60 aromatischen Ringatomen, die mit einem oder mehreren Resten $R^6$ substituiert sein kann, oder eine Diarylaminogruppe, Diheteroarylaminogruppe oder Arylheteroarylaminogruppe mit 10 bis 40 aromatischen Ringatomen, welche mit einem oder mehreren Resten $R^6$ substituiert sein kann, wobei $R^6$ mit einem oder mehreren weiteren Resten $R^6$ ein

mono- oder polycyclisches aliphatisches Ringsystem bilden kann.

In einer weiteren Ausführungsform der erfindungsgemäßen organischen Moleküle besteht die chemische Einheit D aus einer Struktur der Formel IIb, der Formel IIb-2, der Formel IIb-3 oder der der Formel IIb-4:

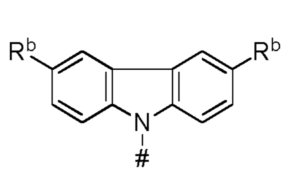
Formel IIb

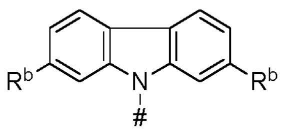
Formel IIb-2

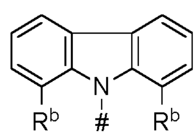
Formel IIb-3

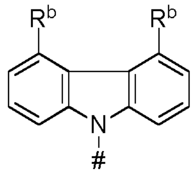
Formel IIb-4

wobei gilt

$R^b$ ist bei jedem Auftreten gleich oder verschieden $N(R^6)_2$, OH, $Si(R^6)_3$, $B(OR^6)_2$, $OSO_2R^6$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils mit einem oder mehreren Resten $R^6$ substituiert sein kann, oder eine Aryloxy- oder Heteroaryloxygruppe mit 5 bis 60 aromatischen Ringatomen, die mit einem oder mehreren Resten $R^6$ substituiert sein kann, oder eine Diarylaminogruppe, Diheteroarylaminogruppe oder Arylheteroarylaminogruppe mit 10 bis 40 aromatischen Ringatomen, welche mit einem oder mehreren Resten $R^6$ substituiert sein kann. Ansonsten gelten die oben genannten Definitionen.

In einer weiteren Ausführungsform der erfindungsgemäßen organischen Moleküle weist die chemische Einheit D eine Struktur der Formel IIc, der Formel IIc-2, der Formel IIc-3 oder der Formel IIc-4 auf oder besteht daraus:

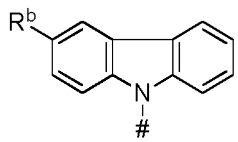
Formel IIc

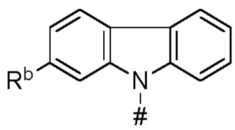
Formel IIc-2

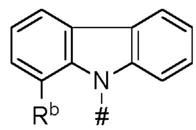
Formel IIc-3

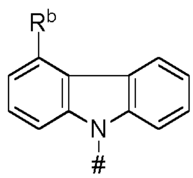
Formel IIc-4

wobei die oben genannten Definitionen gelten.

In einer Ausführungsform ist $R^b$ bei jedem Auftreten gleich oder verschieden $N(R^6)_2$, OH, $Si(R^6)_3$, $B(OR^6)_2$, $OSO_2R^6$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können; oder eine Aryloxy- oder Heteroaryloxygruppe mit 5 bis 60 aromatischen Ringatomen, die mit einem oder mehreren Resten $R^6$ substituiert sein kann, oder eine Diarylaminogruppe, Diheteroarylaminogruppe oder Arylheteroarylaminogruppe mit 10 bis 40 aromatischen Ringatomen, welche mit einem oder mehreren Resten $R^6$ substituiert sein kann, wobei $R^6$ mit einem oder mehreren weiteren Resten $R^6$ ein mono- oder polycyclisches aliphatisches Ringsystem bilden kann. Ansonsten gelten die oben genannten Definitionen.

In einer weiteren Ausführungsform der erfindungsgemäßen organischen Moleküle ist $R^b$ bei jedem Auftreten unabhängig voneinander ausgewählt aus der Gruppe bestehend aus Me, $^i$Pr, $^t$Bu, CN, $CF_3$, Ph, das jeweils mit einem oder mehreren Resten ausgewählt aus Me, $^i$Pr, $^t$Bu, CN, $CF_3$ oder Ph substituiert sein kann, Pyridinyl, das jeweils mit einem oder mehreren Resten ausgewählt aus Me, $^i$Pr, $^t$Bu, CN, $CF_3$ oder Ph substituiert sein kann, Pyrimidinyl, das jeweils mit einem oder mehreren Resten ausgewählt aus Me, $^i$Pr, $^t$Bu, CN, $CF_3$ oder Ph substituiert sein kann, Carbazolyl, das jeweils mit einem oder mehreren Resten ausgewählt aus Me, $^i$Pr, $^t$Bu, CN, $CF_3$ oder Ph substituiert sein kann, Triazinyl, das jeweils mit einem oder mehreren Resten ausgewählt aus Me, $^i$Pr, $^t$Bu, CN, $CF_3$ oder Ph substituiert sein kann, und $N(Ph)_2$.

Im Folgenden sind beispielhaft Ausführungsformen der chemischen Gruppe D gezeigt (erfindungsgemäß sind

solche zweite chemische Einheiten D, die eine Struktur der Formel IIa haben bzw. bei denen Z eine direkte Bindung ist):

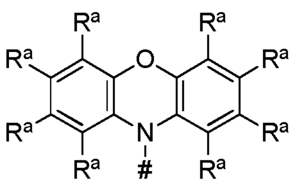
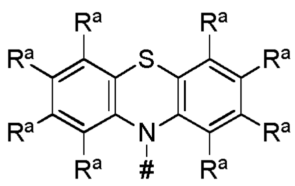
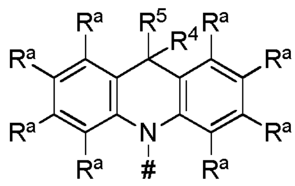
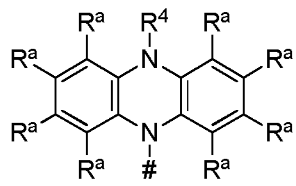

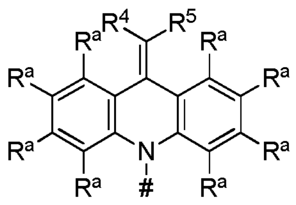
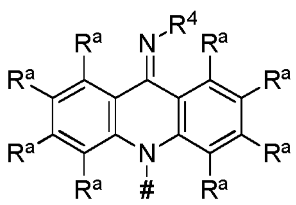
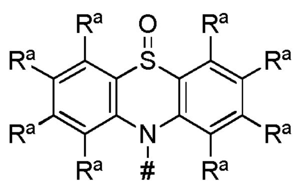

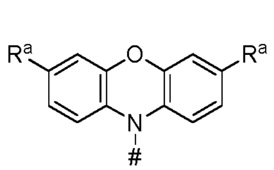
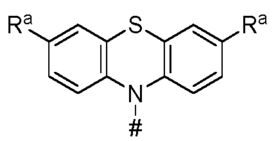
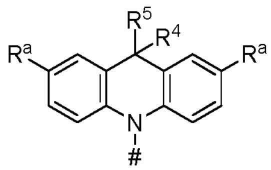
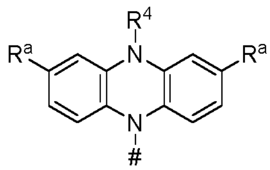

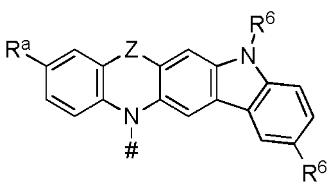
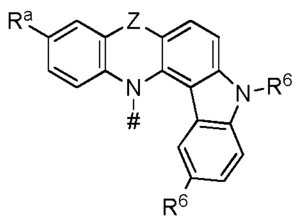
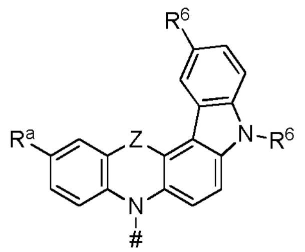

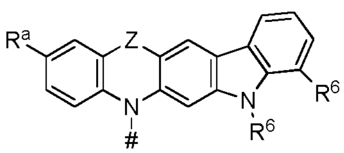
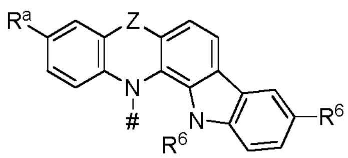
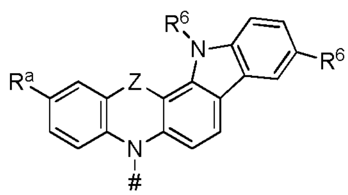

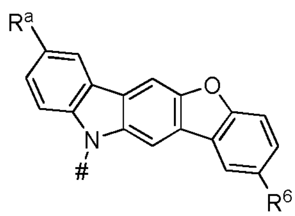
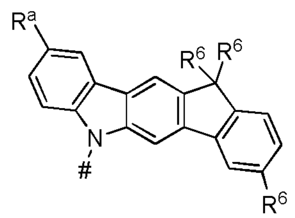
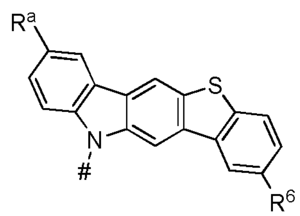

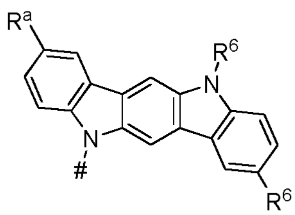

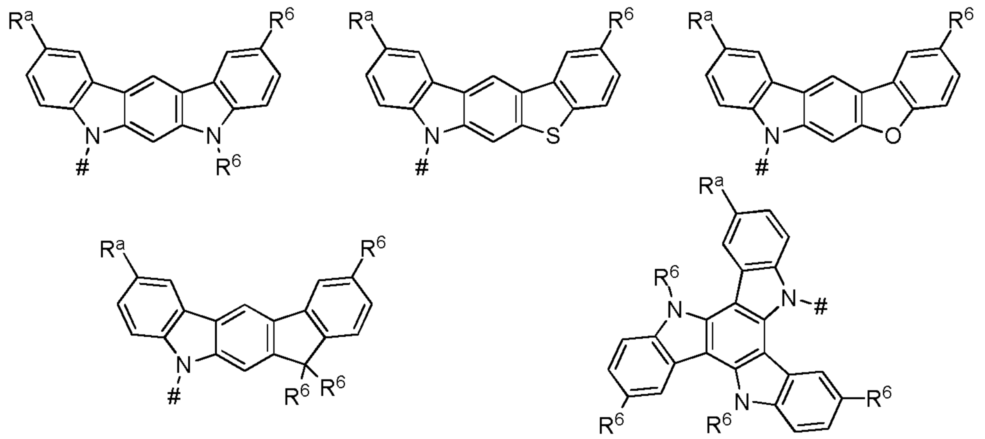

wobei für #, Z, $R^a$, $R^4$, $R^5$ und $R^6$ die oben genannten Definitionen gelten. In einer Ausführungsform ist der Rest $R^6$ bei jedem Auftreten gleich oder verschieden ausgewählt aus der Gruppe bestehend aus H, Methyl, Ethyl, Phenyl und Mesityl. In einer Ausführungsform ist $R^a$ bei jedem Auftreten gleich oder verschieden ausgewählt aus der Gruppe bestehend aus H, Methyl (Me), i-Propyl ($CH(CH_3)_2$) ($^iPr$), t-Butyl ($^tBu$), Phenyl (Ph), CN, $CF_3$ und Diphenylamin ($NPh_2$).

In einer Ausführungsform weisen die erfindungsgemäßen organischen Moleküle eine Struktur der Formel III auf:

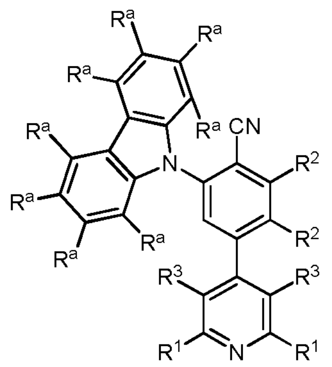

Formel III

wobei die oben genannten Definitionen gelten.

In einer weiteren Ausführungsform weisen die erfindungsgemäßen organischen Moleküle eine Struktur der Formel IIIa auf:

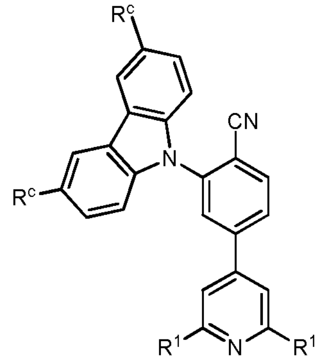

Formel IIIa

wobei

$R^c$ bei jedem Auftreten unabhängig voneinander ausgewählt aus der Gruppe bestehend aus Me, $^iPr$, $^tBu$, CN, $CF_3$, Ph, das jeweils mit einem oder mehreren Resten ausgewählt aus Me, $^iPr$, $^tBu$, CN, $CF_3$ oder Ph substituiert sein kann,

Pyridinyl, das jeweils mit einem oder mehreren Resten ausgewählt aus Me, $^{i}$Pr, $^{t}$Bu, CN, $CF_3$ oder Ph substituiert sein kann, Pyrimidinyl, das jeweils mit einem oder mehreren Resten ausgewählt aus Me, $^{i}$Pr, $^{t}$Bu, CN, $CF_3$ oder Ph substituiert sein kann, Carbazolyl, das jeweils mit einem oder mehreren Resten ausgewählt aus Me, $^{i}$Pr, $^{t}$Bu, CN, $CF_3$ oder Ph substituiert sein kann, Triazinyl, das jeweils mit einem oder mehreren Resten ausgewählt aus Me, $^{i}$Pr, $^{t}$Bu, CN, $CF_3$ oder Ph substituiert sein kann, und $N(Ph)_2$ ist und ansonsten die oben genannten Definitionen gelten.

In einer weiteren Ausführungsform weisen die erfindungsgemäßen organischen Moleküle eine Struktur der Formel IIIb auf:

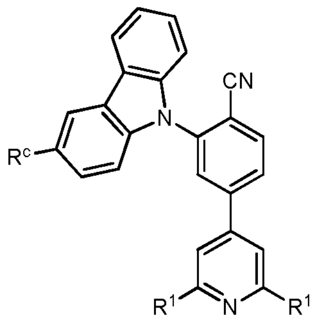

Formel IIIb

wobei die oben genannten Definitionen gelten.

In einer weiteren Ausführungsform weisen die erfindungsgemäßen organischen Moleküle eine Struktur der Formel IIIc auf:

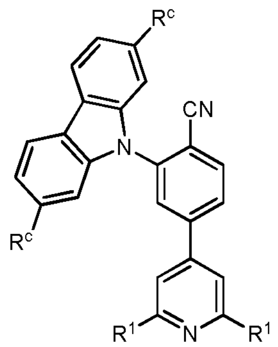

Formel IIIc

wobei die oben genannten Definitionen gelten.

In einer weiteren Ausführungsform weisen die erfindungsgemäßen organischen Moleküle eine Struktur der Formel IIId auf:

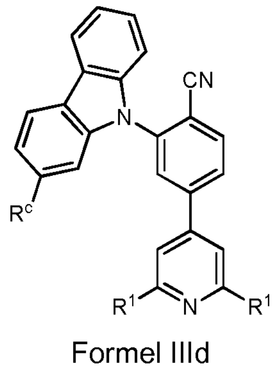

Formel IIId

wobei die oben genannten Definitionen gelten.

In einer weiteren Ausführungsform weisen die erfindungsgemäßen organischen Moleküle eine Struktur der

Formel IIIe auf:

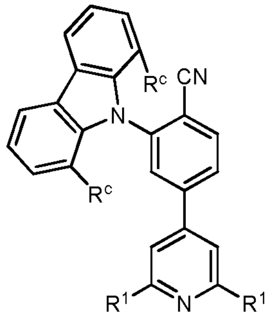

Formel IIIe

wobei die oben genannten Definitionen gelten.

In einer weiteren Ausführungsform weisen die erfindungsgemäßen organischen Moleküle eine Struktur der Formel IIIf auf:

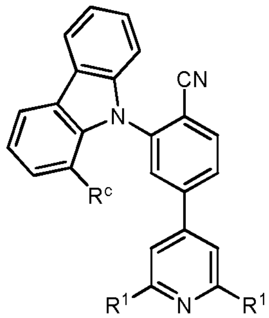

Formel IIIf

wobei die oben genannten Definitionen gelten.

In einer weiteren Ausführungsform weisen die erfindungsgemäßen organischen Moleküle eine Struktur der Formel IIIg auf:

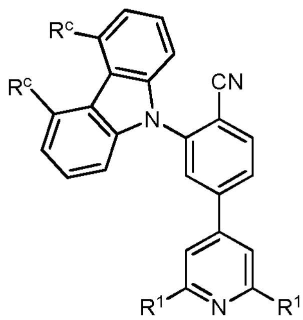

Formel IIIg

wobei die oben genannten Definitionen gelten.

In einer weiteren Ausführungsform weisen die erfindungsgemäßen organischen Moleküle eine Struktur der Formel IIIh auf:

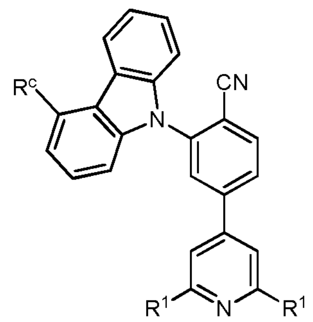

Formel IIIh

wobei die oben genannten Definitionen gelten.

In einer Ausführungsform weisen die erfindungsgemäßen organischen Moleküle eine Struktur der Formel IV auf:

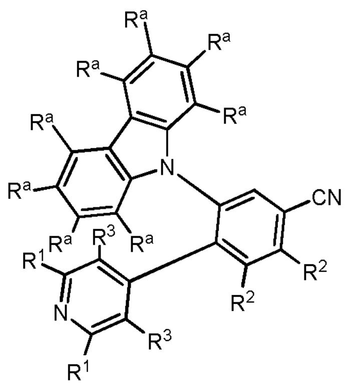

Formel IV

wobei die oben genannten Definitionen gelten.

In einer weiteren Ausführungsform weisen die erfindungsgemäßen organischen Moleküle eine Struktur der Formel IVa auf:

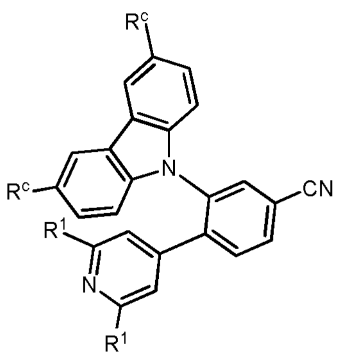

Formel IVa

wobei die oben genannten Definitionen gelten.

In einer weiteren Ausführungsform weisen die erfindungsgemäßen organischen Moleküle eine Struktur der Formel IVb auf:

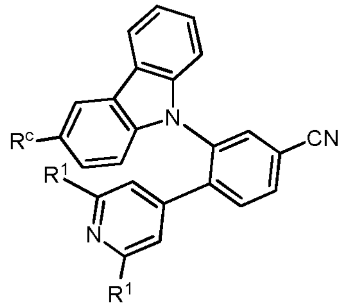

Formel IVb

wobei die oben genannten Definitionen gelten.

In einer weiteren Ausführungsform weisen die erfindungsgemäßen organischen Moleküle eine Struktur der Formel IVc auf:

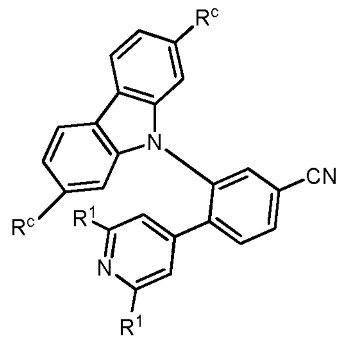

Formel IVc

wobei die oben genannten Definitionen gelten.

In einer weiteren Ausführungsform weisen die erfindungsgemäßen organischen Moleküle eine Struktur der Formel IVd auf:

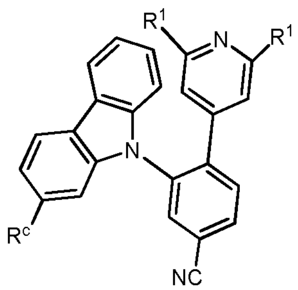

Formel IVd

wobei die oben genannten Definitionen gelten.

In einer weiteren Ausführungsform weisen die erfindungsgemäßen organischen Moleküle eine Struktur der Formel IVe auf:

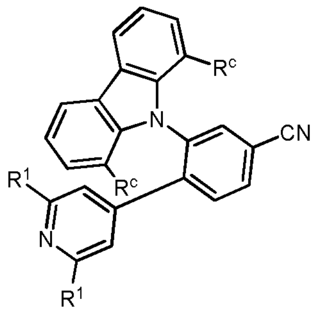

Formel IVe

wobei die oben genannten Definitionen gelten.

In einer weiteren Ausführungsform weisen die erfindungsgemäßen organischen Moleküle eine Struktur der Formel IVf auf:

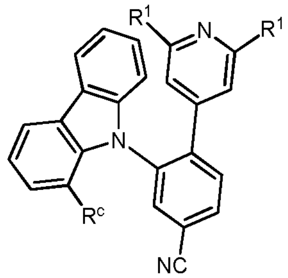

Formel IVf

wobei die oben genannten Definitionen gelten.

In einer weiteren Ausführungsform weisen die erfindungsgemäßen organischen Moleküle eine Struktur der Formel IVg auf:

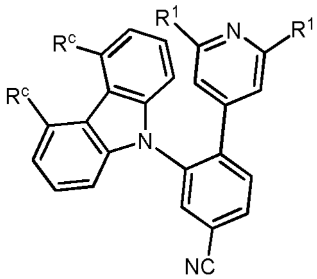

Formel IVg

wobei die oben genannten Definitionen gelten.

In einer weiteren Ausführungsform weisen die erfindungsgemäßen organischen Moleküle eine Struktur der Formel IVh auf:

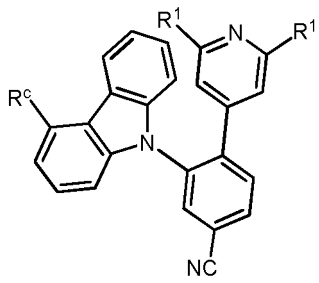

Formel IVh

wobei die oben genannten Definitionen gelten.

In einer Ausführungsform ist $R^c$ bei jedem Auftreten unabhängig voneinander ausgewählt aus der Gruppe bestehend aus Me, $^i$Pr, $^t$Bu, CN, $CF_3$, Ph, das jeweils mit einem oder mehreren Resten ausgewählt aus Me, $^i$Pr, $^t$Bu, CN, $CF_3$ oder Ph substituiert sein kann, Pyridinyl, das jeweils mit einem oder mehreren Resten ausgewählt aus Me, $^i$Pr, $^t$Bu, CN, $CF_3$ oder Ph substituiert sein kann, Pyrimidinyl, das jeweils mit einem oder mehreren Resten ausgewählt aus Me, $^i$Pr, $^t$Bu, CN, $CF_3$ oder Ph substituiert sein kann, Triazinyl, das jeweils mit einem oder mehreren Resten ausgewählt aus Me, $^i$Pr, $^t$Bu, CN, $CF_3$ oder Ph substituiert sein kann, und $N(Ph)_2$ ist und ansonsten die oben genannten Definitionen gelten

In einer Ausführungsform ist $R^c$ ist bei jedem Auftreten unabhängig voneinander ausgewählt aus der Gruppe bestehend aus Me, $^i$Pr, $^t$Bu, Ph, das jeweils mit einem oder mehreren Resten ausgewählt aus Me, $^i$Pr, $^t$Bu, CN, $CF_3$ oder Ph substituiert sein kann, und Carbazolyl, das jeweils mit einem oder mehreren Resten ausgewählt aus Me, $^i$Pr, $^t$Bu, oder Ph substituiert sein kann.

In einer weiteren Ausführungsform ist $R^c$ ist bei jedem Auftreten unabhängig voneinander ausgewählt aus der Gruppe bestehend aus Me, $^i$Pr, $^t$Bu und Ph, das jeweils mit einem oder mehreren Resten ausgewählt aus Me, $^i$Pr, $^t$Bu, CN, $CF_3$ oder Ph substituiert sein kann.

Im Sinne dieser Erfindung enthält eine Arylgruppe 6 bis 60 aromatische Ringatome; eine Heteroarylgruppe enthält 5 bis 60 aromatische Ringatome, von denen mindestens eines ein Heteroatom darstellt. Die Heteroatome sind insbesondere N, O und/oder S. Werden in der Beschreibung bestimmter Ausführungsformen der Erfindung andere, von der genannten Definition abweichende Definitionen angegeben, beispielsweise bezüglich der Zahl der aromatischen Ringatome oder der enthaltenen Heteroatome, so gelten diese.

Unter einer Arylgruppe bzw. Heteroarylgruppe wird ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol oder Kombinationen der genannten Gruppen.

Unter einer cyclischen Alkyl-, Alkoxy- oder Thioalkoxygruppe wird hier eine monocyclische, eine bicyclische oder eine polycyclische Gruppe verstanden.

Im Rahmen der vorliegenden Erfindung werden unter einer $C_1$- bis $C_{40}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neoPentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluor-methyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-0ctyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Eine Ausführungsform der Erfindung betrifft organische Moleküle, welche einen $\Delta E(S_1\text{-}T_1)$-Wert zwischen dem untersten angeregten Singulett ($S_1$)- und dem darunter liegenden Triplett ($T_1$)-Zustand von nicht höher als 5000 $cm^{-1}$, insbesondere nicht höher als 3000 $cm^{-1}$, oder nicht höher als 1500 $cm^{-1}$ oder 1000 $cm^{-1}$ aufweisen und/oder eine Emissionslebensdauer von höchstens 150 $\mu$s, insbesondere von höchstens 100 $\mu$s, von höchsten 50 $\mu$s, oder von höchstens 10 $\mu$s aufweisen und/oder eine Hauptemissionsbande mit einer Halbwertsbreite kleiner als 0,55 eV, insbesondere kleiner als 0,50 eV, kleiner als 0,48 eV, oder kleiner als 0,45 eV aufweisen.

Eine Ausführungsform der Erfindung betrifft organische Moleküle, welche ein Emissionsmaximum bei zwischen 420 und 500 nm, bevorzugter zwischen 430 und 480 nm, noch bevorzugter zwischen 450 und 470 nm aufweisen.

Insbesondere weisen die Moleküle einen "blue material index" (BMI), den Quotienten aus der PLQY (in %) und ihrer $CIE_y$-Farbkoordinate des von dem erfindungsgemäßen Molekül emittierten Lichts, von größer 150, insbesondere von größer 200, von größer 250 oder von größer 300 auf.

In einem weiteren Aspekt betrifft die Erfindung ein Verfahren zur Herstellung eines erfindungsgemäßen organischen Moleküls der hier beschriebenen Art (mit einer eventuellen Folgeumsetzung), wobei eine in 3,5-Position $R^1$-substituierte und in 2,6-Position $R^3$-substituierte 4-Pyridinboronsäure oder ein entsprechender in 3,5-Position $R^1$-substituierter und in 2,6-Position $R^3$-substituierter 4-Pyridinboronsäureester als Edukt eingesetzt wird.

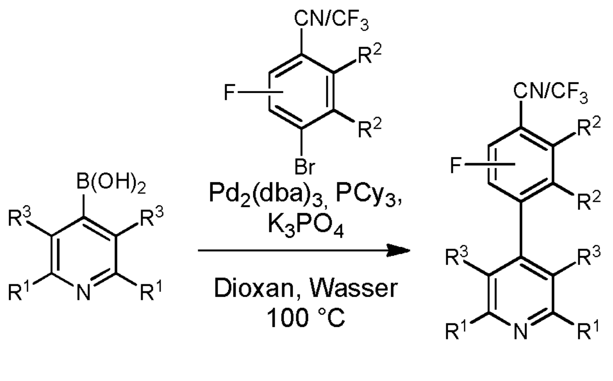


**E1**

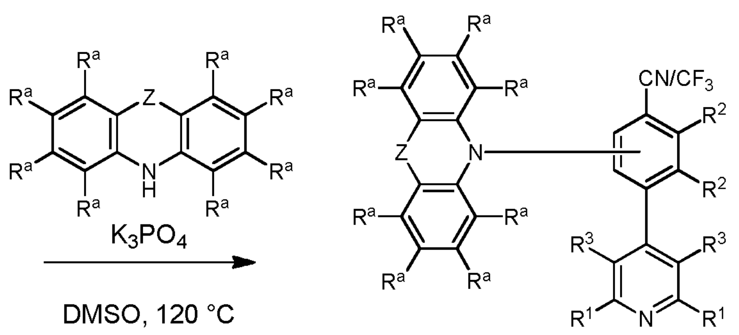


In einer Ausführungsform wird eine in 3,5-Position $R^1$-substituierte und in 2,6-Position $R^3$-substituierte 4-Pyridinboronsäure oder ein entsprechender in 3,5-Position $R^1$-substituierter und in 2,6-Position $R^3$-substituierter 4-Pyridinboronsäureester als Edukt mit einem Bromfluorbenzonitril in einer Palladium-katalysierten Kreuzkupplungsreaktion umgesetzt. Hierbei können erfindungsgemäß 4-Brom-2-fluorbenzonitril, 4-Brom-3-fluorbenzonitril, 4-Brom-2-fluorbenzotrifluorid oder 4-Brom-3-fluorbenzotrifluorid eingesetzt werden. Das Produkt wird durch Deprotonierung des entsprechenden Amins und anschließender nukleophiler Substitution der zwei Fluorgruppen erhalten. Hierbei wird ein Stickstoffheterozyklus im Sinne einer nukleophilen aromatischen Substitution mit einem Edukt E1 umgesetzt. Typische Bedingungen beinhalten die Verwendung einer Base wie beispielweise tribasisches Kaliumphosphat oder Natriumhydrid in einem aprotischen polaren Lösungsmittel wie beispielweise Dimetylsulfoxid (DMSO) oder N,N-Dimethylformamid (DMF).

In einem weiteren Aspekt betrifft die Erfindung die Verwendung der organischen Moleküle als lumineszierender Emitter oder als Hostmaterial in einer organischen optoelektronischen Vorrichtung, insbesondere wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

In einem weiteren Aspekt betrifft die Erfindung eine Zusammensetzung aufweisend oder bestehend aus:

(a) mindestens einem erfindungsgemäßen organischen Molekül, insbesondere als Emitter und/oder Host, und
(b) mindestens ein, d. h. ein oder mehrere Emitter- und/oder Hostmaterialien, die von dem erfindungsgemäßen organischen Molekül verschiedenen ist bzw. sind und
(c) optional einem oder mehreren Farbstoffen und/ oder einem oder mehreren organischen Lösungsmitteln.

In einer Ausführungsform besteht die erfindungsgemäße Zusammensetzung aus einem erfindungsgemäßen organischen Molekül und einem oder mehreren Hostmaterialien. Das oder die Hostmaterialen weisen insbesondere Triplett ($T_1$)- und Singulett ($S_1$)- Energieniveaus auf, die energetisch höher liegen als die Triplett ($T_1$)- und Singulett ($S_1$)- Energieniveaus des erfindungsgemäßen organischen Moleküls. In einer Ausführungsform weist die Zusammensetzung neben dem erfindungsgemäßen organischen Molekül ein elektronendominantes und ein lochdominantes Hostmaterial auf. Das höchste besetzte Orbital (HOMO) und das niedrigste unbesetzte Orbital (LUMO) des lochdominanten Hostmaterials liegen energetisch insbesondere höher als das des elektronendominanten Hostmaterials. Das HOMO des lochdominanten Hostmaterials liegt energetisch unter dem HOMO des erfindungsgemäßen organischen Moleküls, während das LUMO des elektronendominanten Hostmaterials energetisch über dem LUMO des erfindungsgemäßen organischen Moleküls liegt. Um Exciplex-Formation zwischen Emitter und Hostmaterial oder Hostmaterialien zu vermeiden, sollten die Materialien so gewählt sein, dass die Energieabstände zwischen den jeweiligen Orbitalen gering sind. Der Abstand zwischen dem LUMO des elektronendominanten Hostmaterials und dem LUMO des erfindungsgemäßen organischen Moleküls beträgt insbesondere weniger als 0,5 eV, bevorzugt weniger als 0,3 eV, noch bevorzugter weniger als 0,2 eV. Der Abstand zwischen dem HOMO des lochdominanten Hostmaterials und dem HOMO des erfindungsgemäßen organischen Moleküls beträgt insbesondere weniger als 0,5 eV, bevorzugt weniger als 0,3 eV, noch bevorzugter weniger als 0,2 eV.

In einem weiteren Aspekt betrifft die Erfindung eine organische optoelektronische Vorrichtung, die ein erfindungsgemäßes organisches Molekül oder eine erfindungsgemäße Zusammensetzung aufweist. Die organische optoelektronische Vorrichtung ist insbesondere ausgeformt als eine Vorrichtung ausgewählt aus der Gruppe bestehend aus organischer lichtemittierender Diode (OLED); lichtemittierender elektrochemischer Zelle; OLED-Sensor, insbesondere nicht hermetisch nach außen abgeschirmten Gas- und Dampf-Sensoren; organischer Diode; organischer Solarzelle; organischem Transistor; organischem Feldeffekttransistor; organischem Laser und Down-Konversion-Element.

Eine organische optoelektronische Vorrichtung aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein erfindungsgemäßes organisches Molekül aufweist, stellt einen weitere Ausführungsform der Erfindung dar.

In einer Ausführungsform handelt es sich bei der optoelektronischen Vorrichtung um eine OLED. Eine typische OLED weist beispielsweise folgenden Schichtaufbau auf:

1. Substrat (Trägermaterial)

2. Anode
3. Lochinjektionsschicht (hole injection layer, HIL)
4. Lochtransportschicht (hole transport layer, HTL)
5. Elektronenblockierschicht (electron blocking layer, EBL)
6. Emitterschicht (emitting layer, EML)
7. Lochblockierschicht (hole blocking layer, HBL)
8. Elektronenleitschicht (electron transport layer, ETL)
9. Elektroneninjektionsschicht (electron injection layer, EIL)
10. Kathode.

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

Gemäß einer Ausführungsform ist mindestens eine Elektrode des organischen Bauelements transluzent ausgebildet. Hier wird mit "transluzent" eine Schicht bezeichnet, die durchlässig für sichtbares Licht ist. Dabei kann die transluzente Schicht klar durchscheinend, also transparent, oder zumindest teilweise Licht absorbierend und/oder teilweise Licht streuend sein, so dass die transluzente Schicht beispielsweise auch diffus oder milchig durchscheinend sein kann. Insbesondere ist eine hier als transluzent bezeichnete Schicht möglichst transparent ausgebildet, so dass insbesondere die Absorption von Licht so gering wie möglich ist.

Gemäß einer weiteren Ausführungsform weist das organische Bauelement, insbesondere eine OLED, einen invertierten Aufbau auf. Der invertierte Aufbau zeichnet sich dadurch aus, dass sich die Kathode auf dem Substrat befindet und die anderen Schichten entsprechend invertiert aufgebracht werden:

1. Substrat (Trägermaterial)
2. Kathode
3. Elektroneninjektionsschicht (electron injection layer, EIL)
4. Elektronenleitschicht (electron transport layer, ETL)
5. Lochblockierschicht (hole blocking layer, HBL)
6. Emissionsschicht bzw. Emitterschicht (emitting layer, EML)
7. Elektronenblockierschicht (electron blocking layer, EBL)
8. Lochtransportschicht (hole transport layer, HTL)
9. Lochinjektionsschicht (hole injection layer, HIL)
10. Anode

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

In einer Ausführungsform wird bei der invertierten OLED die Anodenschicht des typischen Aufbaus, z.B. eine ITO-Schicht (Indium-Zinn-Oxid), als Kathode geschaltet.

Gemäß einer weiteren Ausführungsform weist das organische Bauelement, insbesondere eine OLED, einen gestapelten Aufbau auf. Hierbei werden die einzelnen OLEDs übereinander und nicht wie üblich nebeneinander angeordnet. Durch einen gestapelten Aufbau kann die Erzeugung von Mischlicht ermöglicht werden. Beispielsweise kann dieser Aufbau bei der Erzeugung von weißem Licht eingesetzt werden, für dessen Erzeugung das gesamte sichtbare Spektrum typischerweise durch die Kombination des emittierten Lichts von blauen, grünen und roten Emittern abgebildet wird. Weiterhin können bei praktisch gleicher Effizienz und identischer Leuchtdichte signifikant längere Lebensdauern im Vergleich zu üblichen OLEDs erzielt werden. Für den gestapelten Aufbau wird optional eine sogenannte Ladungserzeugungsschicht (charge generation layer, CGL) zwischen zwei OLEDs eingesetzt. Diese besteht aus einer n-dotierten und einem p-dotierten Schicht, wobei die n-dotierte Schicht typischerweise näher an der Anode aufgebracht wird.

In einer Ausführungsform - einer sogenannten Tandem-OLED - treten zwei oder mehr Emissionsschichten zwischen Anode und Kathode auf. In einer Ausführungsform sind drei Emissionsschichten übereinander angeordnet, wobei eine Emissionsschicht rotes Licht emittiert, eine Emissionsschicht grünes Licht emittiert und eine Emissionsschicht blaues Licht emittiert und optional weitere Ladungserzeugungs-, Blockier- oder Transportschichten zwischen den einzelnen Emissionsschichten aufgebracht sind. In einer weiteren Ausführungsform werden die jeweiligen Emissionsschichten direkt angrenzend aufgebracht. In einer weiteren Ausführungsform befindet sich jeweils eine Ladungserzeugungsschicht zwischen den Emissionsschichten. Weiterhin können in einer OLED direkt angrenzende und durch Ladungserzeugungsschichten getrennte Emissionsschichten kombiniert werden.

Über den Elektroden und den organischen Schichten kann weiterhin noch eine Verkapselung angeordnet sein. Die Verkapselung kann beispielsweise in Form eines Glasdeckels oder in Form einer Dünnschichtverkapselung ausgeführt sein.

Als Trägermaterial der optoelektronischen Vorrichtung kann beispielsweise Glas, Quarz, Kunststoff, Metall,

ein Siliziumwafer oder jedes andere geeignete feste oder flexible, optional durchsichtige Material dienen. Das Trägermaterial kann beispielsweise ein oder mehrere Materialien in Form einer Schicht, einer Folie, einer Platte oder einem Laminat aufweisen.

Als Anode der optoelektronischen Vorrichtung können beispielsweise transparente leitende Metalloxide wie beispielsweise ITO (Indium-Zinn-Oxid), Zinkoxid, Zinnoxid, Cadmiumoxid, Titanoxid, Indiumoxid oder Aluminiumzinkoxid (AZO), $Zn_2SnO_4$, $CdSnO_3$, $ZnSnO_3$, $MgIn_2O_4$, $GaInO_3$, $Zn_2In_2O_5$ oder $In_4Sn_3O_{12}$ oder Mischungen unterschiedlicher transparenter leitender Oxide dienen.

Als Materialien einer HIL können beispielsweise PEDOT:PSS (Poly-3,4-ethylendioxythiophen:Polystyrolsulfonsäure), PEDOT (Poly-3,4-ethylendioxythiophen), m-MTDATA (4,4',4"-Tris[phenyl(m-tolyl)amino]triphenylamin), Spiro-TAD (2,2',7,7'-Tetrakis(N,N-diphenylamino)-9,9-spirobifluoren), DNTPD (4,4'-Bis[N-[4-{N,N-bis(3-methylphenyl)amino}phenyl]-N-phenylamino]biphenyl), NPB (N,N'-Bis-(1-naphthalenyl)-N,N'-bis-phenyl-(1,1'-biphenyl)-4,4'-diamin), NPNPB (N,N'-Diphenyl-N,N'-di-[4-(N,N-diphenyl-amino)phenyl]benzol), MeO-TPD (N,N,N',N'-Tetrakis(4-methoxyphenyl)benzol), HAT-CN (1,4,5,8,9,11-Hexaazatriphenylen-hexacarbonitril) oder Spiro-NPD (N,N'-diphenyl-N,N'-Bis-(1-naphthyl)-9,9'-spirobifluorene-2,7-diamin) dienen. Beispielhaft ist die Schichtdicke 10-80 nm. Desweiteren können kleine Moleküle verwendet werden (z. B. Kupfer-Phthalocyanin (CuPc z. B. 10 nm dick)) oder Metalloxide wie beispielhaft $MoO_3$, $V_2O_5$.

Als Materialien einer HTL können tertiäre Amine, Carbazolderivate, mit Polystyrolsulfonsäure dotiertes Polyethylendioxythiophen, mit Camphersulfonsäure dotiertes Polyanilin poly-TPD (Poly(4-butylphenyl-diphenyl-amin)), [alpha]-NPD (Poly(4-butylphenyl-diphenyl-amin)), TAPC (4,4'-Cyclohexyliden-bis[*N*,*N*-bis(4-methylphenyl)benzenamin]), TCTA (Tris(4-carbazoyl-9-ylphenyl)amin), 2-TNATA (4,4',4"-Tris[2-naphthyl(phenyl)amino]triphenylamin), Spiro-TAD, DNTPD, NPB, NPNPB, MeO-TPD, HAT-CN oder TrisPcz (9,9'-Diphenyl-6-(9-phenyl-9H-carbazol-3-yl)-9H,9'H-3,3'-bicarbazol) dienen. Beispielhaft ist die Schichtdicke 10 nm bis 100 nm.

Die HTL kann eine p-dotierte Schicht aufweisen, die einen anorganischen oder organischen Dotierstoff in einer organischen löcherleitenden Matrix aufweist. Als anorganischer Dotierstoff können beispielsweise Übergangsmetalloxide wie etwa Vanadiumoxid, Molybdänoxid oder Wolframoxid genutzt werden. Als organische Dotierstoffe können beispielsweise Tetrafluorotetracyanoquinodimethan (F4-TCNQ), Kupfer-Pentafluorobenzoat (Cu(I)pFBz) oder Übergangsmetallkomplexe verwendet werden. Beispielhaft ist die Schichtdicke 10 nm bis 100 nm.

Als Materialien einer Elektronenblockierschicht können beispielsweise mCP (1,3-Bis(carbazol-9-yl)benzol), TCTA, 2-TNATA, mCBP (3,3-Di(9H-carbazol-9-yl)biphenyl), tris-Pcz (9,9'-Diphenyl-6-(9-phenyl-9H-carbazol-3-yl)-9H,9'H-3,3'-bicarbazol), CzSi (9-(4-tert-Butylphenyl)-3,6-bis(triphenylsilyl)-9H-carbazol) oder DCB (N,N'-Dicarbazolyl-1,4-dimethylbenzol) dienen. Beispielhaft ist die Schichtdicke 10nm bis 50 nm.

Die Emitter-Schicht EML oder Emissionsschicht besteht aus oder enthält Emittermaterial oder eine Mischung aufweisend mindestens zwei Emittermaterialien und optional ein oder mehreren Hostmaterialien. Geeignete Hostmaterialien sind beispielsweise mCP, TCTA, 2-TNATA, mCBP, CBP (4,4'-Bis-(N-carbazolyl)-biphenyl), Sif87 (Dibenzo[b,d]thiophen-2-yltriphenylsilan), Sif88 (Dibenzo[b,d]thiophen-2-yl)diphenylsilan) oder DPEPO (Bis[2-((oxo)diphenylphosphino)phenyl]ether). Für im Grünen oder im Roten emittierendes Emittermaterial oder einer Mischung aufweisend mindestens zwei Emittermaterialien eignen sich die gängigen Matrixmaterialien wie CBP. Für im Blauen emittierendes Emittermaterial oder einer Mischung aufweisend mindestens zwei Emittermaterialien können UHG-Matrixmaterialien (Ultra-High energy Gap Materialien) (siehe z. B. M.E. Thompson et al., Chem. Mater. 2004, 16, 4743) oder andere sogenannten Wide-Gap-Matrixmaterialien eingesetzt werden. Beispielhaft ist die Schichtdicke 10 nm bis 250 nm.

Die Lochblockierschicht HBL kann beispielsweise BCP (2,9-Dimethyl-4,7-diphenyl-1,10-phenanthrolin = Bathocuproin), Bis-(2-methyl-8-hydroxychinolinato)-(4-phenylphenolato)-aluminium(III) (BAlq), Nbphen (2,9-Bis(naphthalen-2-yl)-4,7-diphenyl-1,10-phenanthrolin), Alq3 (Aluminium-tris(8-hydroxychinolin)), TSPO1 (Diphenyl-4-triphenylsilylphenyl-phosphinoxid) oder TCB/TCP (1,3,5-Tris(N-carbazolyl)benzol/ 1,3,5-tris(carbazol)-9-yl) benzol) aufweisen. Beispielhaft ist die Schichtdicke 10 nm bis 50 nm.

Die Elektronentransportschicht ETL kann beispielsweise Materialien auf Basis von $AlQ_3$, TSPO1, BPyTP2 (2,7-Di(2,2'-bipyridin-5-yl)triphenyl), Sif87, Sif88, BmPyPhB (1,3-Bis[3,5-di(pyridin-3-yl)phenyl]benzol) oder BTB (4,4'-Bis-[2-(4,6-diphenyl-1,3,5-triazinyl)]-1,1'-biphenyl) aufweisen. Beispielhaft ist die Schichtdicke 10 nm bis 200 nm.

Als Materialien einer dünnen Elektroneninjektionsschicht EIL können beispielsweise CsF, LiF, 8-Hydroxyquinolinolatolithium (Liq), $Li_2O$, $BaF_2$, MgO oder NaF verwendet werden.

Als Materialien der Kathodenschicht können Metalle oder Legierungen dienen, beispielsweise Al, Al > AlF, Ag, Pt, Au, Mg, Ag:Mg. Typische Schichtdicken betragen 100 nm bis 200 nm. Insbesondere werden ein oder mehrere Metalle verwendet, die stabil an der Luft sind und/oder die selbstpassivierend, beispielsweise durch Ausbildung einer dünnen schützenden Oxidschicht, sind.

Als Materialien zu Verkapselung sind beispielsweise Aluminiumoxid, Vanadiumoxid, Zinkoxid, Zirkoniumoxid, Titanoxid, Hafniumoxid, Lanthanoxid, Tantaloxid geeignet.

In einer Ausführungsform der erfindungsgemäßen organischen optoelektronischen Vorrichtung ist das erfindungsgemäße organische Molekül als Emissionsmaterial in einer lichtemittierenden Schicht EML eingesetzt, wobei es

entweder als Reinschicht oder in Kombination mit einem oder mehreren Hostmaterialien eingesetzt ist.

Eine Ausführungsform der Erfindung betrifft organische optoelektronische Vorrichtungen, welche eine externe Quanteneffizienz (EQE) bei 1000 cd/$m^2$ von größer 5 %, insbesondere von größer 8 %, insbesondere von größer 10 %, oder von größer 13 %, oder von größer 16 % und insbesondere von größer 20 % und/oder ein Emissionsmaximum bei einer Wellenlänge zwischen 420 nm und 500 nm, insbesondere zwischen 430 nm und 490 nm, oder zwischen 440 nm und 480 nm und insbesondere zwischen 450 nm und 470 nm und/oder einen LT80 Wert bei 500 cd/$m^2$ von größer 30 h, insbesondere von größer 70 h, oder von größer 100 h, oder von größer 150 h und insbesondere von größer 200 h aufweisen.

Der Massenanteil des erfindungsgemäßen organischen Moleküls an der Emitter-Schicht EML beträgt in einer weiteren Ausführungsform in einer lichtemittierenden Schicht in optischen Licht emittierenden Vorrichtungen, insbesondere in OLEDs, zwischen 1 % und 80 %. In einer Ausführungsform der erfindungsgemäßen organischen optoelektronischen Vorrichtung ist die lichtemittierende Schicht auf ein Substrat aufgebracht, wobei bevorzugt eine Anode und eine Kathode auf das Substrat aufgebracht sind und die lichtemittierende Schicht zwischen Anode und Kathode aufgebracht ist.

Die lichtemittierende Schicht kann in einer Ausführungsform ausschließlich ein erfindungsgemäßes organisches Molekül in 100 % Konzentration aufweisen, wobei die Anode und die Kathode auf das Substrat aufgebracht sind, und die lichtemittierende Schicht zwischen Anode und Kathode aufgebracht ist.

In einer Ausführungsform der erfindungsgemäßen organischen optoelektronischen Vorrichtung sind eine löcher- und elektroneninjizierende Schicht zwischen Anode und Kathode, und eine löcher- und elektronentransportierende Schicht zwischen löcher- und elektroneninjizierende Schicht, und die lichtemittierende Schicht zwischen löcher- und elektronentransportierender Schicht aufgebracht.

Die organische optoelektronische Vorrichtung weist in einer weiteren Ausführungsform der Erfindung auf: ein Substrat, eine Anode, eine Kathode und mindestens je eine löcher- und elektroneninjizierende Schicht, und mindestens je eine löcher- und elektronentransportierende Schicht, und mindestens eine lichtemittierende Schicht, die erfindungsgemäßes organisches Molekül und ein oder mehrere Hostmaterialen aufweist, deren Triplett ($T_1$)- und Singulett ($S_1$)-Energieniveaus energetisch höher liegen als die Triplett ($T_1$)- und Singulett ($S_1$)-Energieniveaus des organischen Moleküls, wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierende Schicht aufgebracht ist, und die lichtemittierende Schicht zwischen löcher- und elektronentransportierende Schicht aufgebracht ist.

In einem weiteren Aspekt betrifft die Erfindung ein Verfahren zur Herstellung eines optoelektronischen Bauelements. Dabei wird ein erfindungsgemäßes organisches Molekül verwendet.

In einer Ausführungsform umfasst das Herstellungsverfahren die Verarbeitung des erfindungsgemäßen organischen Moleküls mittels eines Vakuumverdampfungsverfahrens oder aus einer Lösung.

Erfindungsgemäß ist auch ein Verfahren zur Herstellung einer erfindungsgemäßen optoelektronischen Vorrichtung, bei dem mindestens eine Schicht der optoelektronischen Vorrichtung

- mit einem Sublimationsverfahren beschichtet wird,
- mit einem OVPD (Organic Vapor Phase Deposition) Verfahren beschichtet wird,
- mit einer Trägergassublimation beschichtet wird, und/oder
- aus Lösung oder mit einem Druckverfahren hergestellt wird.

Bei der Herstellung der erfindungsgemäßen optoelektronischen Vorrichtung werden bekannte Verfahren eingesetzt. Generell werden die Schichten einzeln auf ein geeignetes Substrat in aufeinanderfolgenden Abscheideverfahrensschritten aufgebracht. Bei der Gasphasenabscheidung können die gebräuchlichen Methoden, wie thermische Verdampfung, chemische Gasphasenabscheidung (CVD), physikalische Gasphasenabscheidung (PVD) angewendet werden. Für active matrix OLED (AMOLED) Displays erfolgt die Abscheidung auf eine AMOLED Backplane als Substrat.

Alternativ können Schichten aus Lösungen oder Dispersionen in geeigneten Lösungsmitteln aufgebracht werden. Beispielhafte geeignete Beschichtungsverfahren sind Rotationsbeschichtung (spin-coating), Tauchbeschichtung (dip-coating) und Strahldruckmethoden. Die einzelnen Schichten können erfindungsgemäß sowohl über dasselbe als auch über jeweils verschiedene Beschichtungsmethoden hergestellt werden.

**Beispiele**

Allgemeines Syntheseschema I

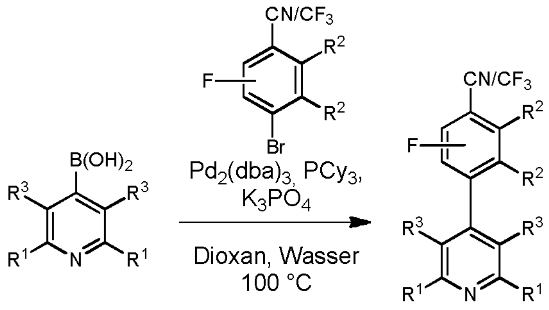


**E1**

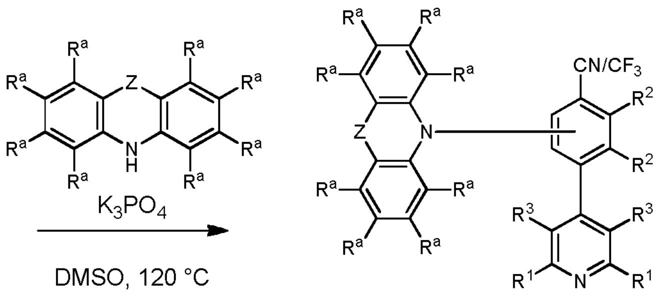


*Allgemeine Synthesevorschrift **AAV1:***

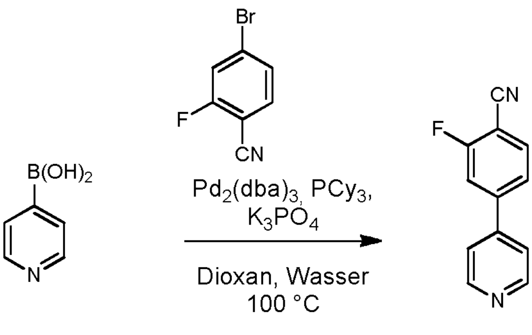


**Z1**

4-Pyridinboronsäure, (1,50 Äquivalente), 4-Brom-2-fluorbenzonitril (1,00 Äquivalente), $Pd_2(dba)_3$ (0,03 Äquivalente), Tricyclohexylphosphin ($PCy_3$) (0,08 Äquivalente) und tribasisches Kaliumphosphat (2,50 Äquivalente) werden unter Stickstoff in einem Dioxan/Wasser-Gemisch (Verhältnis 4:1) bei 100 °C für 16 h gerührt. Anschließend wird die Reaktionsmischung in gesättigte Natriumchlorid-Lösung gegeben und zweimal mit Ethylacetat extrahiert. Die vereinigten organischen Phasen werden mit gesättigter Natriumchlorid-Lösung gewaschen, über MgSO4 getrocknet und das Lösemittel entfernt. Das Produkt wird über wenig Kieselgel filtriert und danach umkristallisiert. Das Produkt wird als Feststoff erhalten. Erfindungsgemäß kann statt einer Boronsäure auch ein entsprechender Boronsäureester verwendet werden.

*Allgemeine Synthesevorschrift **AAV2**:*

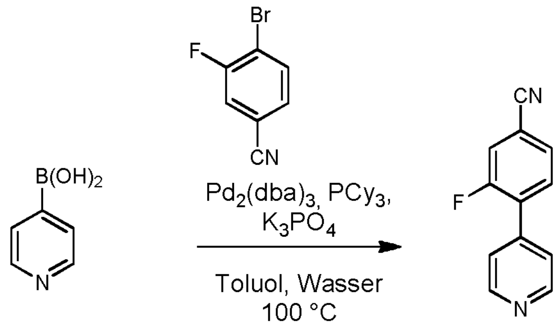


Z2

Die Synthese von **Z2** erfolgt analog ***AAV1***, wobei 4-Pyridinboronsäure mit 4-Brom-3-fluorbenzonitril umgesetzt wird.

*Allgemeine Synthesevorschrift* ***AAV3*****:**

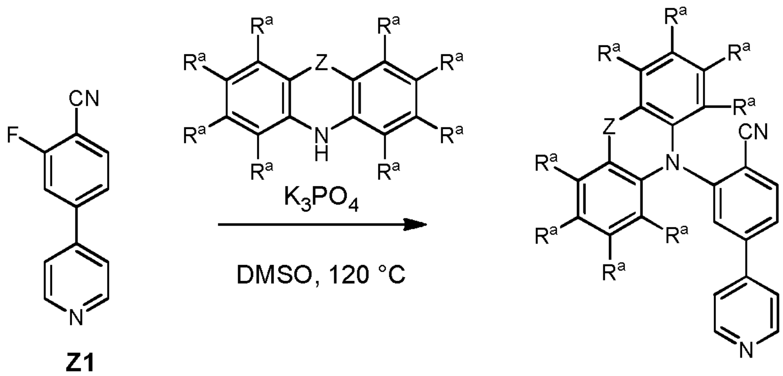


Z1

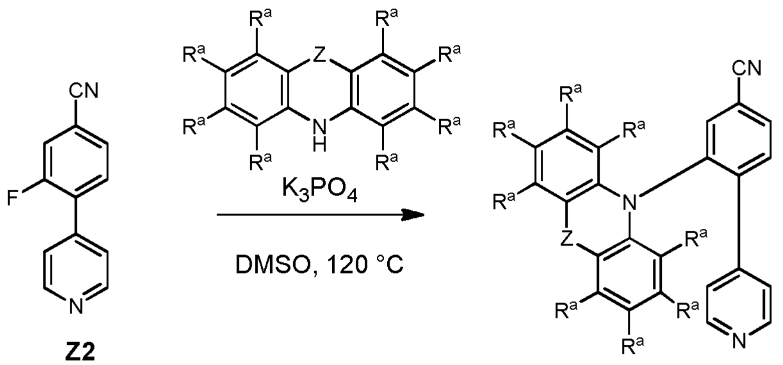


Z2

**Z1** oder **Z2** (jeweils 1,00 Äquivalente), das entsprechende Donor-Molekül D-H (2,00 Äquivalente) und tribasisches Kaliumphosphat (4,00 Äquivalente) werden unter Stickstoff in DMSO suspendiert und bei 120 °C gerührt (16 h). Anschließend wird die Reaktionsmischung in gesättigte Natriumchlorid-Lösung gegeben und dreimal mit Dichlormethan extrahiert. Die vereinigten organischen Phasen werden zweimal mit gesättigter Natriumchlorid-Lösung gewaschen, getrocknet über Magnesiumsulfat und das Lösemittel anschließend entfernt. Das Rohprodukt wird schließlich durch Umkristallisation aus Toluol oder über Flashchromatographie gereinigt. Das Produkt wird als Feststoff erhalten.

Im speziellen entspricht D-H einem 3,6-substituierten Carbazol (z. B. 3,6-Dimethylcarbazol, 3,6-Diphenylcarbazol, 3,6-Di-tert-butylcarbazol), einem 2,7-substituierten Carbazol (z. B. 2,7-Dimethylcarbazol, 2,7-Diphenylcarbazol, 2,7-Di-tert-butylcarbazol), einem 1,8-substituierten Carbazol (z. B. 1,8-Dimethylcarbazol, 1,8-Diphenylcarbazol, 1,8-Di-tert-butylcarbazol), einem 1-substituierten Carbazol (z. B. 1-Methylcarbazol, 1-Phenylcarbazol, 1-tert-Butylcarbazol), einem 2-substituierten Carbazol (z. B. 2-Methylcarbazol, 2-Phenylcarbazol, 2-tert-Butylcarbazol) oder einem 3-substituierten Carbazol (z. B. 3-Methylcarbazol, 3-Phenylcarbazol, 3-tert-Butylcarbazol).

**Photophysikalische Messungen**

*Vorbehandlung von optischen Gläsern*

Alle Gläser (Küvetten und Substrate aus Quarzglas, Durchmesser: 1 cm) wurden nach jeder Benutzung gereinigt: Je dreimaliges Spülen mit Dichlormethan, Aceton, Ethanol, demineralisiertem Wasser, Einlegen in 5 % Hellmanex-Lösung für 24 h, gründliches Ausspülen mit demineralisiertem Wasser. Zum Trocknen wurden die optischen Gläser mit Stickstoff abgeblasen.

*Probenvorbereitung, Film: Spin-Coating*

Gerät: Spin150, SPS euro.

Die Probenkonzentration entsprach 10 mg/ml, angesetzt in Toluol oder Chlorbenzol. Programm: 1) 3 s bei 400 U/min; 2) 20 s bei 1000 U/min bei 1000 Upm/ s. 3) 10 s bei 4000 U/min bei 1000 Upm/s. Die Filme wurden nach dem Beschichten für 1 min bei 70 °C an Luft auf einer Präzisionsheizplatte von LHG getrocknet.

Photolumineszenzspektroskopie und TCSPC

Steady-state Emissionsspektroskopie wurde mit einem Fluoreszenzspektrometer der Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer "zeit-korrelierten Einphotonzähl" (*Time-correlated single-photon counting,* TCSPC)-Option. Emissions- und Anregungsspektren wurden korrigiert durch Standardkorrekturkurven.

Die Emissionsabklingzeiten wurden ebenfalls auf diesem System gemessen unter Verwendung der TCSPC-Methode mit dem FM-2013 Zubehör und einem TCSPC-Hub von Horiba Yvon Jobin. Anregungsquellen:

NanoLED 370 (Wellenlänge: 371 nm, Pulsdauer: 1,1 ns)
NanoLED 290 (Wellenlänge: 294 nm, Pulsdauer: <1 ns)
SpectraLED 310 (Wellenlänge: 314 nm)
SpectraLED 355 (Wellenlänge: 355 nm).

Die Auswertung (exponentielles Fitten) erfolgte mit dem Softwarepaket DataStation und der DAS 6 Auswertungssoftware. Der Fit wurde über die Chi-Quadrat-Methode angegeben

$$c^2 = \sum_{k=1}^{i} \frac{(e_i - o_i)^2}{e_i}$$

mit $e_i$: Durch den Fit vorhergesagte Größe und $o_i$: gemessenen Größe.

Quanteneffizienzbestimmung

Die Messung der Photolumineszenzquantenausbeute (PLQY) erfolgte mittels eines *Absolute PL Quantum Yield Measurement C9920-03G*-Systems der *Hamamatsu Photonics.* Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- *(back thinned-)* CCD-Chip mit 1024 x 122 Pixeln (Größe 24 x 24 μm) verbunden ist. Die Auswertung der Quanteneffizienz und der CIE-Koordinaten erfolgte mit Hilfe der Software U6039-05 Version 3.6.0. Das Emissionsmaximum wird in nm, die Quantenausbeute Φ in % und die CIE-Farbkoordinaten als x,y-Werte angegeben.

Die Photolumineszenzquantenausbeute wurde nach folgendem Protokoll bestimmt:

1) Durchführung der Qualitätssicherung: Als Referenzmaterial dient Anthracene in Ethanol mit bekannter Konzentration.
2) Ermitteln der Anregungswellenlänge: Es wurde zuerst das Absorbtionsmaximum des organischen Moleküls bestimmt und mit diesem angeregt.

3) Durchführung der Probenmessung:
Es wurde von entgasten Lösungen und Filmen unter Stickstoff-Atmosphäre die absolute Quantenausbeute bestimmt.

Die Berechnung erfolgte systemintern nach folgender Gleichung:

$$\Phi_{PL} = \frac{n_{photon}, emittiert}{n_{photon}, absorbiert} = \frac{\int \frac{\lambda}{hc}\left[Int_{emittiert}^{Probe}(\lambda) - Int_{absorbiert}^{Probe}(\lambda)\right]d\lambda}{\int \frac{\lambda}{hc}\left[Int_{emittiert}^{Referenz}(\lambda) - Int_{absorbiert}^{Referenz}(\lambda)\right]d\lambda}$$

mit der Photonenzahl $n_{photon}$ und der Intensität Int.

Herstellung und Charakterisierung von organischen Elektrolumineszenzvorrichtungen aus der Gasphase

Mit den erfindungsgemäßen organischen Molekülen können OLED-Devices mittels Vakuum-Sublimationstechnik erstellt werden. Enthält eine Schicht mehrere Komponenten, so ist das Verhältnis dieser in Massenprozent angegeben.

Diese noch nicht optimierten OLEDs können standardmäßig charakterisiert werden; hierfür werden die Elektrolumineszenzspektren, die externe Quanteneffizienz (gemessen in %) in Abhängigkeit von der Helligkeit, berechnet aus dem von der Fotodiode detektiertem Licht, und dem Strom aufgenommen. Aus dem zeitlichen Verlauf der Elektrolumineszenzspektren kann die Lebensdauer der OLEDs bestimmt werden. Der angegebene LT50-Wert entspricht hierbei der Zeit, bei der die Leuchtdichte auf 50 % des Startwertes abgefallen ist. Analog entspricht der LT70-Wert der Zeit, bei der die Leuchtdichte auf 70 % des Startwertes abgefallen ist.

Die angegebenen Werte ergeben sich aus dem Durchschnitt der verschiedenen Pixel einer OLED. Die abgebildeten Spektren zeigen jeweils eine Messreihe eines Pixels.

HPLC-MS:

HPLC-MS Spektroskopie wurde mit einer HPLC-Anlage der Firma Agilent (1100er Serie) mit einem angeschlossenen MS-Detektor (Thermo LTQ XL) gemessen. Für die HPLC wurde eine Eclipse Plus C18 Säule von Agilent mit einer Partikelgröße von 3,5 µm, einer Länge von 150 mm und einem Innendurchmesser von 4,6 mm eingesetzt. Es wurde ohne Vorsäule und bei Raumtemperatur mit den Lösemitteln Acetonitril, Wasser und Tetrahydrofuran in diesen Konzentrationen gearbeitet:

| | | |
|---|---|---|
| Lösemittel A: | $H_2O$ (90 %) | MeCN (10 %) |
| Lösemittel B: | $H_2O$ (10 %) | MeCN (90 %) |
| Lösemittel C: | THF (100 %) | |

Es wurde mit einem Einspritzvolumen von 15 µL und einer Konzentration von 10 µg/mL mit diesem Gradienten gearbeitet:

| Fluss [ml/min] | Zeit [min] | A[%] | B[%] | C[%] | Druck [bar] |
|---|---|---|---|---|---|
| 0,3 | 0 | 80 | 20 | - | 115 |
| 0,3 | 5 | 80 | 20 | - | 115 |
| 0,3 | 14 | 0 | 90 | 10 | 65 |
| 0,3 | 25 | 0 | 90 | 10 | 65 |
| 0,3 | 26 | 80 | 20 | - | 115 |
| 0,3 | 33 | 80 | 20 | - | 115 |

Die Ionisation der Probe erfolgt durch APCI (atmospheric pressure chemical ionization).

**Beispiel 1**

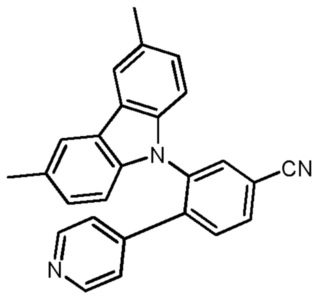

Beispiel 1 wurde nach AAV2 (Ausbeute 18%) und AAV3 hergestellt (Ausbeute 57%).

1H-NMR (500 MHz, CDCl3): σ = 8.32, (dd, 2H, Ar-H), 7.93 (dd, 1H, Ar-H), 7.89 (d, 1H, Ar-H), 7.83 (bs, 2H, Ar-H), 7.80 (d, 1H, Ar-H), 2.50 (s, 6H, $CH_3$) ppm.
$R_f$ = 0,07 (Cyclohexan/Ethylacetat 5:1).
Figur 1 zeigt das Emissionsspektrum von Beispiel **1** (10 % in PMMA). Das Emissionsmaximum liegt bei 459 nm. Die Photolumineszenzquantenausbeute (PLQY) beträgt 67% und die Halbwertsbreite beträgt 0,47 eV.

**Beispiel 2**

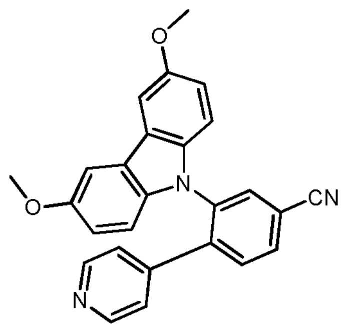

Beispiel **2** wurde nach AAV2 (Ausbeute 18%) und AAV3 (Ausbeute 70%) hergestellt.

$R_f$ = 0,02 (Cyclohexan/Ethylacetat 5:1).
Figur 2 zeigt das Emissionsspektrum von Beispiel **2** (10 % in PMMA). Das Emissionsmaximum liegt bei 497 nm. Die Photolumineszenzquantenausbeute (PLQY) beträgt 51 % und die Halbwertsbreite beträgt 0,48 eV.

**Beispiel 3**

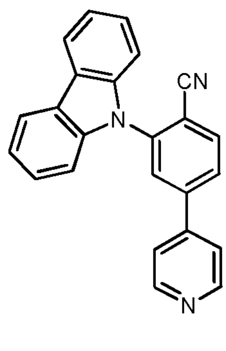

Beispiel **3** wurde nach AAV1 (Ausbeute 50%) und AAV3 (Ausbeute 67%) hergestellt.

$^1$H-NMR (500 MHz, $CDCl_3$): d = 8.80 (d, 2H, Ar-H), 8.20 (d, 2H, Ar-H), 8.13 (d, 1H, Ar-H), 7.91-7.94 (m, 2H, Ar-H), 7.67-7.68 (m, 2H, Ar-H), 7.47-7.50 (m, 2H, Ar-H), 7.38-7.41 (m, 2H, Ar-H), 7.27 (s, 1H, Ar-H) ppm.

Figur 3 zeigt das Emissionsspektrum von Beispiel **3** (10 % in PMMA). Das Emissionsmaximum liegt bei 445 nm. Die Photolumineszenzquantenausbeute (PLQY) beträgt 34 % und die Halbwertsbreite beträgt 0,59 eV.

**Beispiel 4**

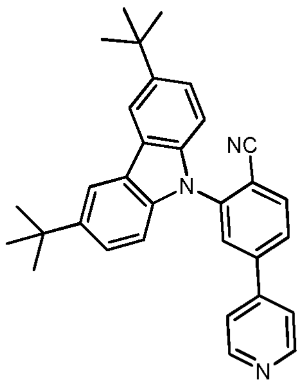

Beispiel **3** wurde nach AAV1 (Ausbeute 50%) und AAV3 (Ausbeute 68%) hergestellt.

$^1$H-NMR (500 MHz, $CDCl_3$): d = 7.78 (d, 2H, Ar-H), 8.18 (d, 2H, Ar-H), 8.11 (d, 1H, Ar-H), 7.86-7.89 (m, 2H, Ar-H), 7.63 (d, 2H, Ar-H), 7.52 (dd, 2H, Ar-H), 7.22 (d, 2H, Ar-H), 1.50 (s, 18H, $CH_3$) ppm.

Figur 4 zeigt das Emissionsspektrum von Beispiel **4** (10 % in PMMA). Das Emissionsmaximum liegt bei 466 nm. Die Photolumineszenzquantenausbeute (PLQY) beträgt 64 % und die Halbwertsbreite beträgt 0,48 eV.

**Beispiel 5**

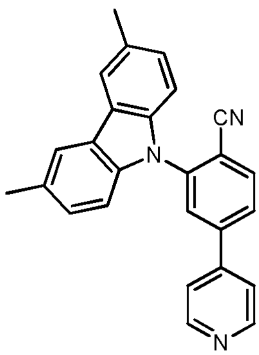

Beispiel **5** wurde nach AAV1 (Ausbeute 50%) und AAV3 (Ausbeute 53%) hergestellt.

$^1$H-NMR (500 MHz, $CDCl_3$): d = 8.78 (d, 2H, Ar-H), 8.09 (d, 1H, Ar-H), 7.94 (s, 2H, Ar-H), 7.90 (d, 1H, Ar-H), 7.86 (dd, 1H, Ar-H), 7.63 (m, 2H, Ar-H), 7.27 (m, 2H, Ar-H), 7.17 (d, 2H, Ar-H), 2.57 (s, 6H, Ar-H) ppm.

Figur 5 zeigt das Emissionsspektrum von Beispiel **5** (10 % in PMMA). Das Emissionsmaximum liegt bei 468 nm. Die Photolumineszenzquantenausbeute (PLQY) beträgt 60 % und die Halbwertsbreite beträgt 0,47 eV.

**Figuren**

Es zeigen:

Figur 1 Emissionsspektrum von Beispiel **1** (10 % in PMMA).
Figur 2 Emissionsspektrum von Beispiel **2** (10 % in PMMA).
Figur 3 Emissionsspektrum von Beispiel **3** (10 % in PMMA).
Figur 4 Emissionsspektrum von Beispiel **4** (10 % in PMMA).
Figur 5 Emissionsspektrum von Beispiel **5** (10 % in PMMA).

**Patentansprüche**

1. Organisches Molekül aufweisend

- eine erste chemische Einheit bestehend aus einer Struktur gemäß Formel I

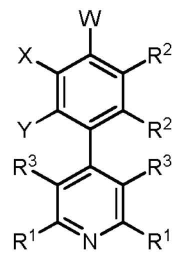

Formel I

und
- eine zweite chemische Einheit D bestehend aus einer Struktur gemäß Formel II,

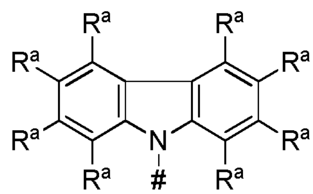

Formel IIa

wobei die erste chemische Einheit über eine Einfachbindung mit der zweiten chemischen Einheit D verknüpft ist; mit
X ist H oder Anknüpfungspunkt der Einfachbindung zwischen der ersten chemischen Einheit und der zweiten chemischen Einheit D;
Y ist H oder Anknüpfungspunkt der Einfachbindung zwischen der ersten chemischen Einheit und der zweiten chemischen Einheit D;
W ist CN oder $CF_3$;
# kennzeichnet den Anknüpfungspunkt der Einfachbindung zwischen der zweiten chemischen Einheit D und der ersten chemischen Einheit;
$R^1$, $R^2$, und $R^3$ ist bei jedem Auftreten gleich oder verschieden ausgewählt aus der Gruppe bestehend aus H, Deuterium,
einer linearen Alkylgruppe mit 1 bis 5 C-Atomen, einer linearen Alkenyl- oder Alkinylgruppe mit 2 bis 8 C-Atomen, einer verzweigten oder cyclischen Alkyl-, Alkenyl- oder Alkinylgruppe mit 3 bis 10 C-Atomen, wobei in den zuvor genannten Gruppen ein oder mehrere H-Atome durch Deuterium ersetzt sein können,
einem aromatischen Ringsystem mit 5 bis 15 aromatischen Ringatomen, das mit einem oder mehreren Resten $R^7$ substituiert sein kann, und
einem heteroaromatischen Ringsystem mit 5 bis 15 aromatischen Ringatomen, das mit einem oder mehreren Resten $R^7$ substituiert sein kann;

$R^a$, $R^4$ und $R^5$ ist bei jedem Auftreten gleich oder verschieden ausgewählt aus der Gruppe bestehend aus H, Deuterium, $N(R^6)_2$, OH, $Si(R^6)_3$, $B(OR^6)_2$, $OSO_2R^6$, $CF_3$, CN, F, Br, I, einer linearen Alkyl-, Alkoxy- oder Thioalkoxygruppe mit 1 bis 40 C-Atomen, die jeweils mit einem oder mehreren Resten $R^6$ substituiert sein kann und wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können; einer linearen Alkenyl- oder Alkinylgruppe mit 2 bis 40 C-Atomen, die jeweils mit einem oder mehreren Resten $R^6$ substituiert sein kann und wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können; einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann und wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
einem aromatischen Ringsystem mit 5 bis 60 aromatischen Ringatomen, das mit einem oder mehreren Resten $R^6$ substituiert sein kann,
einem heteroaromatischen Ringsystem mit 5 bis 60 aromatischen Ringatomen, das mit einem oder mehreren Resten $R^6$ substituiert sein kann,
einer Aryloxy- oder Heteroaryloxygruppe mit 5 bis 60 aromatischen Ringatomen, die mit einem oder mehreren Resten $R^6$ substituiert sein kann, und
einer Diarylaminogruppe, Diheteroarylaminogruppe oder Arylheteroarylaminogruppe mit 10 bis 40 aromatischen Ringatomen, welche mit einem oder mehreren Resten $R^6$ substituiert sein kann;
$R^6$ ist bei jedem Auftreten gleich oder verschieden ausgewählt aus der Gruppe bestehend aus H, Deuterium, $N(R^7)_2$, OH, $Si(R^7)_3$, $B(OR^7)_2$, $OSO_2R^7$, $CF_3$, CN, F, Br, I,
einer linearen Alkyl-, Alkoxy- oder Thioalkoxygruppe mit 1 bis 40 C-Atomen, die jeweils mit einem oder mehreren Resten $R^7$ substituiert sein kann und wobei eine oder mehrere nicht benachbarte $CH_2$-Gruppen durch $R^7C=CR^7$, C≡C, $Si(R^7)_2$, $Ge(R^7)_2$, $Sn(R^7)_2$, C=O, C=S, C=Se, $C=NR^7$, $P(=O)(R^7)$, SO, $SO_2$, $NR^7$, O, S oder $CONR^7$ ersetzt sein können und wobei ein oder mehrere H-Atome durch Deuterium, CN, $CF_3$ oder $NO_2$ ersetzt sein können;
einer linearer Alkenyl- oder Alkinylgruppe mit 2 bis 40 C-Atomen, die jeweils mit einem oder mehreren Resten $R^7$ substituiert sein kann und wobei eine oder mehrere nicht benachbarte $CH_2$-Gruppen durch $R^7C=CR^7$, C≡C, $Si(R^7)_2$, $Ge(R^7)_2$, $Sn(R^7)_2$, C=O, C=S, C=Se, $C=NR^7$, $P(=O)(R^7)$, SO, $SO_2$, $NR^7$, O, S oder $CONR^7$ ersetzt sein können und wobei ein oder mehrere H-Atome durch Deuterium, CN, $CF_3$ oder $NO_2$ ersetzt sein können; einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^7$ substituiert sein kann und wobei eine oder mehrere nicht benachbarte $CH_2$-Gruppen durch $R^7C=CR^7$, C≡C, $Si(R^7)_2$, $Ge(R^7)_2$, $Sn(R^7)_2$, C=O, C=S, C=Se, $C=NR^7$, $P(=O)(R^7)$, SO, $SO_2$, $NR^7$, O, S oder $CONR^7$ ersetzt sein können und wobei ein oder mehrere H-Atome durch Deuterium, CN, $CF_3$ oder $NO_2$ ersetzt sein können;
einem aromatischen Ringsystem mit 5 bis 60 aromatischen Ringatomen, das mit einem oder mehreren Resten $R^7$ substituiert sein kann,
einem heteroaromatischen Ringsystem mit 5 bis 60 aromatischen Ringatomen, das mit einem oder mehreren Resten $R^7$ substituiert sein kann,
einer Aryloxy- oder Heteroaryloxygruppe mit 5 bis 60 aromatischen Ringatomen, die mit einem oder mehreren Resten $R^7$ substituiert sein kann, und
einer Diarylaminogruppe, Diheteroarylaminogruppe oder Arylheteroarylaminogruppe mit 10 bis 40 aromatischen Ringatomen, welche mit einem oder mehreren Resten $R^7$ substituiert sein kann;
$R^7$ ist bei jedem Auftreten gleich oder verschieden ausgewählt aus Gruppe bestehen aus H, Deuterium, OH, $CF_3$, CN, F,
einer linearen Alkyl-, Alkoxy- oder Thioalkoxygruppe mit 1 bis 5 C-Atomen, einer linearen Alkenyl- oder Alkinylgruppe mit 2 bis 5 C-Atomen,einer verzweigten oder cyclischen Alkyl-, Alkenyl-, Alkinyl-, Alkoxy- oder Thioalkoxygruppe mit 3 bis 5 C-Atomen, wobei in den vorher genannten Gruppen jeweils ein oder mehrere H-Atome durch Deuterium, CN, $CF_3$ oder $NO_2$ ersetzt sein können;
einem aromatischen oder heteroaromatischen Ringsystem mit 5 bis 60 aromatischen Ringatomen, einer Aryloxy- oder Heteroaryloxygruppe mit 5 bis 60 aromatischen Ringatomen und einer Diarylaminogruppe, Diheteroarylaminogruppe oder Arylheteroarylaminogruppe mit 10 bis 40 aromatischen Ringatomen;
wobei jeder der Reste $R^a$, $R^4$, $R^5$ oder $R^6$ auch mit einem oder mehreren weiteren Resten $R^a$, $R^4$, $R^5$ oder $R^6$ ein mono- oder polycyclisches, aliphatisches, aromatisches und/oder benzoanelliertes Ringsystem bilden kann;
und wobei
genau ein Rest ausgewählt aus der Gruppe bestehend aus X und Y gleich dem Anknüpfungspunkt der Einfachbindung zwischen der ersten chemischen Einheit und der zweiten chemischen Einheit D ist.

**2.** Organisches Molekül nach Anspruch 1, wobei $R^1$, $R^2$ und $R^3$ bei jedem Auftreten gleich oder verschieden H, Methyl oder Phenyl ist.

**3.** Organisches Molekül nach Anspruch 1 oder 2, wobei W gleich CN ist.

**4.** Organisches Molekül nach Anspruch 1 bis 3, wobei die zweite chemische Einheit D aus eine Struktur der Formel IIb besteht:

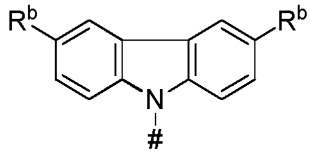

Formel IIb

wobei

$R^b$ bei jedem Auftreten gleich oder verschieden ausgewählt aus der Gruppe bestehend aus $N(R^6)_2$, OH, $Si(R^6)_3$, $B(OR^6)_2$, $OSO_2R^6$, $CF_3$, CN, F, Br, I,
einer linearen Alkyl-, Alkoxy- oder Thioalkoxygruppe mit 1 bis 40 C-Atomen, die jeweils mit einem oder mehreren Resten $R^6$ substituiert sein kann und wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
einer linearen Alkenyl- oder Alkinylgruppe mit 2 bis 40 C-Atomen, die jeweils mit einem oder mehreren Resten $R^6$ substituiert sein kann und wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann und wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
einem aromatischen Ringsystem mit 5 bis 60 aromatischen Ringatomen, das mit einem oder mehreren Resten $R^6$ substituiert sein kann,
einem heteroaromatischen Ringsystem mit 5 bis 60 aromatischen Ringatomen, das mit einem oder mehreren Resten $R^6$ substituiert sein kann,
einer Aryloxy- oder Heteroaryloxygruppe mit 5 bis 60 aromatischen Ringatomen, die mit einem oder mehreren Resten $R^6$ substituiert sein kann, und
einer Diarylaminogruppe, Diheteroarylaminogruppe oder Arylheteroarylaminogruppe mit 10 bis 40 aromatischen Ringatomen, welche mit einem oder mehreren Resten $R^6$ substituiert sein kann, ist;
und für # und $R^6$ die in Anspruch 1 genannten Definitionen gelten.

**5.** Organisches Molekül nach Anspruch 1 bis 3, wobei die zweite chemische Einheit D aus einer Struktur der Formel IIc besteht:

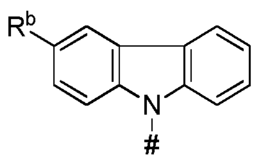

Formel IIc

wobei

$R^b$ bei jedem Auftreten gleich oder verschieden ausgewählt aus der Gruppe bestehend aus $N(R^6)_2$, OH, $Si(R^6)_3$, $B(OR^6)_2$, $OSO_2R^6$, $CF_3$, CN, F, Br, I,
einer linearen Alkyl-, Alkoxy- oder Thioalkoxygruppe mit 1 bis 40 C-Atomen, die jeweils mit einem oder mehreren Resten $R^6$ substituiert sein kann und wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
einer linearen Alkenyl- oder Alkinylgruppe mit 2 bis 40 C-Atomen, die jeweils mit einem oder mehreren Resten $R^6$ substituiert sein kann und wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann und wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
einem aromatischen Ringsystem mit 5 bis 60 aromatischen Ringatomen, das mit einem oder mehreren Resten $R^6$ substituiert sein kann,
einem heteroaromatischen Ringsystem mit 5 bis 60 aromatischen Ringatomen, das mit einem oder mehreren Resten $R^6$ substituiert sein kann,
einer Aryloxy- oder Heteroaryloxygruppe mit 5 bis 60 aromatischen Ringatomen, die mit einem oder mehreren Resten $R^6$ substituiert sein kann, und
einer Diarylaminogruppe, Diheteroarylaminogruppe oder Arylheteroarylaminogruppe mit 10 bis 40 aromatischen Ringatomen, welche mit einem oder mehreren Resten $R^6$ substituiert sein kann, ist;
und im Übrigen die in Anspruch 1 genannte Definitionen gelten.

**6.** Organisches Molekül nach Anspruch 4 oder 5, wobei $R^b$ bei jedem Auftreten gleich oder verschieden Me, $^i$Pr, $^t$Bu, CN, $CF_3$ oder Ph, das jeweils mit einem oder mehreren Resten ausgewählt aus Me, $^i$Pr, $^t$Bu, CN, $CF_3$ und Ph substituiert sein kann, Pyridinyl, das jeweils mit einem oder mehreren Resten ausgewählt aus Me, $^i$Pr, $^t$Bu, CN, $CF_3$ und Ph substituiert sein kann, Pyrimidinyl, das jeweils mit einem oder mehreren Resten ausgewählt aus Me, $^i$Pr, $^t$Bu, CN, $CF_3$ und Ph substituiert sein kann, Triazinyl, das jeweils mit einem oder mehreren Resten ausgewählt aus Me, $^i$Pr, $^t$Bu, CN, $CF_3$ und Ph substituiert sein kann, Carbazolyl, das jeweils mit einem oder mehreren Resten ausgewählt aus Me, $^i$Pr, $^t$Bu, CN, $CF_3$ und Ph substituiert sein kann, oder $N(Ph)_2$ ist.

**7.** Verfahren zur Herstellung eines Organischen Moleküls nach Anspruch 1 bis 6, wobei eine in 3,5-Position $R^1$-substituierte und 2,6-Position $R^3$-substituierte 4-Pyridinboronsäure oder ein entsprechender in 3,5-Position $R^1$-substituierter und 2,6-Position $R^3$-substituierter 4-Pyridinboronsäureester als Edukt eingesetzt wird.

**8.** Verwendung eines organischen Moleküls nach Anspruch 1 bis 6 als lumineszierender Emitter und/oder als Hostmaterial und/oder als Elektronentransportmaterial und/oder als Lochinjektionsmaterial und/oder als Lochblockiermaterial in einer organischen optoelektronischen Vorrichtung.

**9.** Verwendung nach Anspruch 8, wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

**10.** Zusammensetzung aufweisend oder bestehend aus:

(a) mindestens einem organischen Molekül nach einem der Ansprüche 1 bis 6, insbesondere in Form eines Emitters und/oder Hosts, und

(b) einem oder mehrerer Emitter- und/oder Hostmaterialien, die von dem Molekül nach Anspruch 1 bis 6 verschiedenen sind und
(c) optional einem oder mehreren Farbstoffen und/ oder einem oder mehreren Lösungsmitteln.

**11.** Organische optoelektronische Vorrichtung, aufweisend ein organisches Molekül nach Anspruch 1 bis 6 oder eine Zusammensetzung nach Anspruch 10, insbesondere ausgeformt als eine Vorrichtung ausgewählt aus der Gruppe bestehend ausorganischer lichtemittierender Diode (OLED), lichtemittierender elektrochemischer Zelle, OLED-Sensor, insbesondere nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

**12.** Organische optoelektronische Vorrichtung nach Anspruch 11, aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein organisches Molekül nach Anspruch 1 bis 6 oder eine Zusammensetzung nach Anspruch 10 aufweist.

**13.** Verfahren zur Herstellung eines optoelektronischen Bauelements, wobei ein organisches Molekül nach Anspruch 1 bis 6 verwendet wird, insbesondere umfassend die Verarbeitung des organischen Moleküls mittels eines Vakuumverdampfungsverfahrens oder aus einer Lösung.

## Claims

**1.** An organic molecule comprising

- a first chemical unit consisting of a structure according to formula I

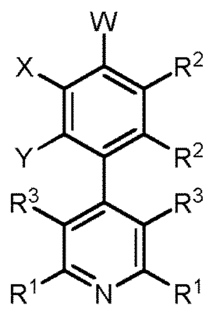

Formula I

and
- a second chemical unit D consisting of a structure according to formula IIa,

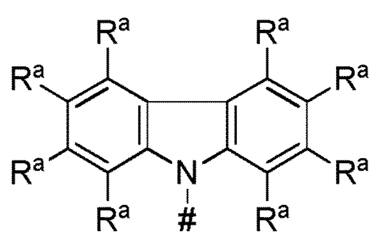

Formula IIa

wherein the first chemical unit is linked to the second chemical unit D by means of a single bond;

where
X is H or connection point of the single bond between the first chemical unit and the second chemical unit D;
Y is H or connection point of the single bond between the first chemical unit and the second chemical unit D;
W is CN or $CF_3$;
# denotes the connection point of the single bond between the second chemical unit D and the first chemical unit;
$R^1$, $R^2$, and $R^3$, identically or differently at each occurrence, is selected from the group consisting of H, deuterium, a linear alkyl group having 1 to 5 C atoms, a linear alkenyl or alkynyl group having 2 to 8 C atoms, a branched or cyclic alkyl, alkenyl or alkynyl group having 3 to 10 C atoms, wherein in the aforementioned groups one or more H atoms may be replaced by deuterium,
an aromatic ring system which has 5 to 15 aromatic ring atoms and which may be substituted with one or more groups $R^7$, and
a heteroaromatic ring system which has 5 to 15 aromatic ring atoms and which may be substituted with one or more groups $R^7$;
$R^a$, $R^4$ and $R^5$, identically or differently at each occurrence, is selected from the group consisting of H, deuterium, $N(R^6)_2$, OH, $Si(R^6)_3$, $B(OR^6)_2$, $OSO_2R^6$, $CF_3$, CN, F, Br, I,
a linear alkyl, alkoxy or thioalkoxy group which has 1 to 40 C atoms and which in each case may be substituted with one or more groups $R^6$, and wherein one or more non-adjacent $CH_2$ groups may be replaced by $R^6C{=}CR^6$, C≡C, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S or $CONR^6$, and wherein one or more H atoms may be replaced by deuterium, CN, $CF_3$ or $NO_2$;
a linear alkenyl or alkynyl group which has 2 to 40 C atoms and which in each case may be substituted with one or more groups $R^6$, and wherein one or more non-adjacent $CH_2$ groups may be replaced by $R^6C{=}CR^6$, C≡C, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S or $CONR^6$, and wherein one or more H atoms may be replaced by deuterium, CN, $CF_3$ or $NO_2$;
a branched or cyclic alkyl, alkenyl, alkynyl, alkoxy or thioalkoxy group which has 3 to 40 C atoms and which in each case may be substituted with one or more groups $R^6$, and wherein one or more non-adjacent $CH_2$ groups may be replaced by $R^6C{=}CR^6$, C≡C, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S or $CONR^6$, and wherein one or more H atoms may be replaced by deuterium, CN, $CF_3$ or $NO_2$;
an aromatic ring system which has 5 to 60 aromatic ring atoms and which may be substituted with one or more groups $R^6$,
a heteroaromatic ring system which has 5 to 60 aromatic ring atoms and which may be substituted with one or more groups $R^6$,
an aryloxy or heteroaryloxy group which has 5 to 60 aromatic ring atoms and which may be substituted with one or more groups $R^6$, and
a diarylamino group, diheteroarylamino group, or arylheteroarylamino group which has 10 to 40 aromatic ring atoms and which may be substituted with one or more groups $R^6$;
$R^6$, identically or differently at each occurrence, is selected from the group consisting of H, deuterium, $N(R^7)_2$, OH, $Si(R^7)_3$, $B(OR^7)_2$, $OSO_2R^7$, $CF_3$, CN, F, Br, I,
a linear alkyl, alkoxy or thioalkoxy group which has 1 to 40 C atoms and which in each case may be substituted with one or more groups $R^7$, and wherein one or more non-adjacent $CH_2$ groups may be replaced by $R^7C{=}CR^7$, C≡C, $Si(R^7)_2$, $Ge(R^7)_2$, $Sn(R^7)_2$, C=O, C=S, C=Se, $C{=}NR^7$, $P({=}O)(R^7)$, SO, $SO_2$, $NR^7$, O, S or $CONR^7$, and wherein one or more H atoms may be replaced by deuterium, CN, $CF_3$ or $NO_2$;
a linear alkenyl or alkynyl group which has 2 to 40 C atoms and which in each case may be substituted with one or more groups $R^7$, and wherein one or more non-adjacent $CH_2$ groups may be replaced by $R^7C{=}CR^7$, C≡C, $Si(R^7)_2$, $Ge(R^7)_2$, $Sn(R^7)_2$, C=O, C=S, C=Se, $C{=}NR^7$, $P({=}O)(R^7)$, SO, $SO_2$, $NR^7$, O, S or $CONR^7$, and wherein one or more H atoms may be replaced by deuterium, CN, $CF_3$ or $NO_2$;
a branched or cyclic alkyl, alkenyl, alkynyl, alkoxy or thioalkoxy group which has 3 to 40 C atoms and which in each case may be substituted with one or more groups $R^7$, and wherein one or more non-adjacent $CH_2$ groups may be replaced by $R^7C{=}CR^7$, C≡C, $Si(R^7)_2$, $Ge(R^7)_2$, $Sn(R^7)_2$, C=O, C=S, C=Se, $C{=}NR^7$, $P({=}O)(R^7)$, SO, $SO_2$, $NR^7$, O, S or $CONR^7$, and wherein one or more H atoms may be replaced by deuterium, CN, $CF_3$ or $NO_2$;
an aromatic ring system which has 5 to 60 aromatic ring atoms and which may be substituted with one or more groups $R^7$,
a heteroaromatic ring system which has 5 to 60 aromatic ring atoms and which may be substituted with one or more groups $R^7$,
an aryloxy or heteroaryloxy group which has 5 to 60 aromatic ring atoms and which may be substituted with one or more groups $R^7$, and
a diarylamino group, diheteroarylamino group, or arylheteroarylamino group which has 10 to 40 aromatic ring atoms and which may be substituted with one or more groups $R^7$;
$R^7$, identically or differently at each occurrence, is selected from the group consisting of H, deuterium, OH, $CF_3$, CN, F,

a linear alkyl, alkoxy or thioalkoxy group having 1 to 5 C atoms, a linear alkenyl or alkynyl group having 2 to 5 C atoms, a branched or cyclic alkyl, alkenyl, alkynyl, alkoxy or thioalkoxy group having 3 to 5 C atoms, wherein in the aforementioned groups in each case one or more H atoms may be replaced by deuterium, CN, $CF_3$ or $NO_2$;
an aromatic ring system having 5 to 60 aromatic ring atoms, an aryloxy or heteroaryloxy group having 5 to 60 aromatic ring atoms, and a diarylamino group, diheteroarylamino group, or arylheteroarylamino group having 10 to 40 aromatic ring atoms;
wherein each of the groups $R^a$, $R^4$, $R^5$ or $R^6$ may also form a mono- or polycyclic, aliphatic, aromatic and/or benzo-annellated ring system together with one or more further groups $R^a$, $R^4$, $R^5$ or $R^6$;
and wherein
precisely one group selected from the group consisting of X and Y is a connection point of the single bond between the first chemical unit and the second chemical unit D.

2. The organic molecule according to claim 1, wherein $R^1$, $R^2$ and $R^3$, identically or differently at each occurrence, H, is methyl or phenyl.

3. The organic molecule according to claim 1 or claim 2, wherein W is CN.

4. The organic molecule according to claims 1 to 3, wherein the second chemical unit D consists of a structure of formula IIb:

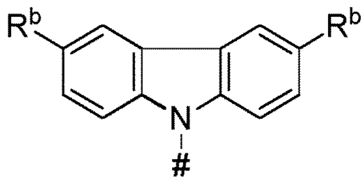

Formula IIb

wherein

$R^b$, identically or differently at each occurrence, is selected from the group consisting of $N(R^6)_2$, OH, $Si(R^6)_3$, $B(OR^6)_2$, $OSO_2R^6$, $CF_3$, CN, F, Br, I,
a linear alkyl, alkoxy or thioalkoxy group which has 1 to 40 C atoms and which in each case may be substituted with one or more groups $R^6$, and wherein one or more non-adjacent $CH_2$ groups may be replaced by $R^6C{=}CR^6$, $C{\equiv}C$, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S or $CONR^6$, and wherein one or more H atoms may be replaced by deuterium, CN, $CF_3$ or $NO_2$;
a linear alkenyl or alkynyl group which has 2 to 40 C atoms and which in each case may be substituted with one or more groups $R^6$, and wherein one or more non-adjacent $CH_2$ groups may be replaced by $R^6C{=}CR^6$, $C{\equiv}C$, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S or $CONR^6$, and wherein one or more H atoms may be replaced by deuterium, CN, $CF_3$ or $NO_2$;
a branched or cyclic alkyl, alkenyl, alkynyl, alkoxy or thioalkoxy group which has 3 to 40 C atoms and which in each case may be substituted with one or more groups $R^6$, and wherein one or more non-adjacent $CH_2$ groups may be replaced by $R^6C{=}CR^6$, $C{\equiv}C$, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S or $CONR^6$, and wherein one or more H atoms may be replaced by deuterium, CN, $CF_3$ or $NO_2$;
an aromatic ring system which has 5 to 60 aromatic ring atoms and which may be substituted with one or more groups $R^6$,
a heteroaromatic ring system which has 5 to 60 aromatic ring atoms and which may be substituted with one or more groups $R^6$,
an aryloxy or heteroaryloxy group which has 5 to 60 aromatic ring atoms and which may be substituted with one or more groups $R^6$, and
a diarylamino group, diheteroarylamino group, or arylheteroarylamino group which has 10 to 40 aromatic ring atoms and which may be substituted with one or more groups $R^6$;
and the definitions as stated in claim 1 apply for # and $R^6$.

5. The organic molecule according to claims 1 to 3, wherein the second chemical unit D consists of a structure of formula IIc:

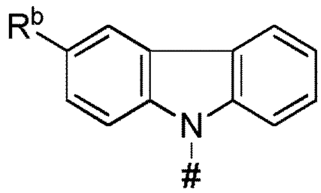

Formula IIc

wherein

$R^b$, identically or differently at each occurrence, is selected from the group consisting of $N(R^6)_2$, OH, $Si(R^6)_3$, $B(OR^6)_2$, $OSO_2R^6$, $CF_3$, CN, F, Br, I,
a linear alkyl, alkoxy or thioalkoxy group which has 1 to 40 C atoms and which in each case may be substituted with one or more groups $R^6$, and wherein one or more non-adjacent $CH_2$ groups may be replaced by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$, and wherein one or more H atoms may be replaced by deuterium, CN, $CF_3$ or $NO_2$;
a linear alkenyl or alkynyl group which has 2 to 40 C atoms and which in each case may be substituted with one or more groups $R^6$, and wherein one or more non-adjacent $CH_2$ groups may be replaced by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$, and wherein one or more H atoms may be replaced by deuterium, CN, $CF_3$ or $NO_2$;
a branched or cyclic alkyl, alkenyl, alkynyl, alkoxy or thioalkoxy group which has 3 to 40 C atoms and which in each case may be substituted with one or more groups $R^6$, and wherein one or more non-adjacent $CH_2$ groups may be replaced by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$, and wherein one or more H atoms may be replaced by deuterium, CN, $CF_3$ or $NO_2$;
an aromatic ring system which has 5 to 60 aromatic ring atoms and which may be substituted with one or more groups $R^6$,
a heteroaromatic ring system which has 5 to 60 aromatic ring atoms and which may be substituted with one or more groups $R^6$,
an aryloxy or heteroaryloxy group which has 5 to 60 aromatic ring atoms and which may be substituted with one or more groups $R^6$, and
a diarylamino group, diheteroarylamino group, or arylheteroarylamino group which has 10 to 40 aromatic ring atoms and which may be substituted with one or more groups $R^6$;
and otherwise the definitions as stated in claim 1 apply.

**6.** The organic molecule according to claim 4 or 5, wherein $R^b$, identically or differently at each occurrence, is Me, $^i$Pr, $^t$Bu, CN, $CF_3$ or Ph, which in each case may be substituted with one or more groups selected from Me, $^i$Pr, $^t$Bu, CN, $CF_3$ and Ph, pyridinyl, which in each case may be substituted with one or more groups selected from Me, $^i$Pr, $^t$Bu, CN, $CF_3$ and Ph, pyrimidinyl, which in each case may be substituted with one or more groups selected from Me, $^i$Pr, $^t$Bu, CN, $CF_3$ and Ph, triazinyl, which in each case may be substituted with one or more groups selected from Me, $^i$Pr, $^t$Bu, CN, $CF_3$ and Ph, carbazolyl, which in each case may be substituted with one or more groups selected from Me, $^i$Pr, $^t$Bu, CN, $CF_3$ and Ph, or $N(Ph)_2$.

**7.** A method for producing an organic molecule according to claims 1 to 6, wherein a 4-pyridinylboronic acid $R^1$-substituted in the 3,5 position and $R^3$-substituted in the 2,6 position or a corresponding 4-pyridinylboronic acid ester $R^1$-substituted in the 3,5 position and $R^3$-substituted in the 2,6 position is used as starting material.

**8.** Use of an organic molecule according to claim 1 to 6 as luminescent emitter and/or as host material and/or as electron transport material and/or as hole injection material and/or as hole-blocking material in an organic optoelectronic device.

**9.** Use according to claim 8, wherein the organic optoelectronic device is selected from the group consisting of:

- organic light-emitting components (OLEDs),
- light-emitting electrochemical cells,
- OLED sensors, in particular in non-hermetically outwardly shielded gas and vapour sensors,
- organic diodes,

- organic solar cells,
- organic transistors,
- organic field-effect transistors,
- organic lasers, and
- down-conversion elements.

**10.** A composition, comprising or consisting of:

(a) at least one organic molecule according to one of claims 1 to 6, in particular in the form of an emitter and/or host, and
(b) one or more emitter and/or host materials which are different from the molecule according to claim 1 to 6, and
(c) optionally one or more dyes and/or one or more solvents.

**11.** An organic optoelectronic device, comprising an organic molecule according to claim 1 to 6 or a composition according to claim 10, in particular formed as a device selected from the group consisting of organic light-emitting diode (OLED), light-emitting electrochemical cell, OLED sensor, in particular non-hermetically outwardly shielded gas and vapour sensors, organic diode, organic solar cell, organic transistor, organic field-effect transistor, organic laser, and down-conversion element.

**12.** The organic optoelectronic device according to claim 11, comprising

- a substrate,
- an anode, and
- a cathode, wherein the anode or the cathode are applied to the substrate, and
- at least one light-emitting layer, which is arranged between the anode and the cathode and which comprises an organic molecule according to claims 1 to 6 or a composition according to claim 10.

**13.** A method for producing an optoelectronic device, wherein an organic molecule according to claims 1 to 6 is used, in particular comprising the processing of the organic molecule by means of a vacuum evaporation process or from a solution.

**Revendications**

**1.** Molécule organique comprenant :

- une première unité chimique constituée par une structure selon la formule I

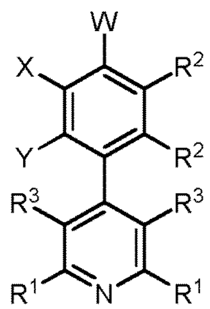

Formule I

et
- une deuxième unité chimique D constituée par une structure selon la formule II

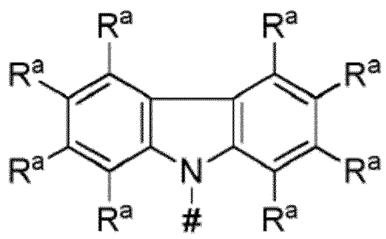

Formule IIa

la première unité chimique étant reliée par une simple liaison avec la deuxième unité chimique D ;
X représentant H ou étant le point de liaison de la simple liaison entre la première unité chimique et la deuxième unité chimique D ;
Y représentant H ou étant le point de liaison de la simple liaison entre la première unité chimique et la deuxième unité chimique D ;
W représentant CN ou $CF_3$ ;
# désignant le point de liaison de la simple liaison entre la deuxième unité chimique D et la première unité chimique ;
$R^1$, $R^2$, et $R^3$ étant choisis à chaque occurrence de manière identique ou différente dans le groupe constitué par H, le deutérium,
un groupe alkyle linéaire de 1 à 5 atomes C, un groupe alcényle ou alcynyle linéaire de 2 à 8 atomes C, un groupe alkyle, alcényle ou alcynyle ramifié ou cyclique de 3 à 10 atomes C, un ou plusieurs atomes H pouvant être remplacés par le deutérium dans les groupes susmentionnés,
un système cyclique aromatique de 5 à 15 atomes de cycle aromatiques, qui peut être substitué avec un ou plusieurs radicaux $R^7$, et
un système cyclique hétéroaromatique de 5 à 15 atomes de cycle aromatiques, qui peut être substitué avec un ou plusieurs radicaux $R^7$ ;
$R^a$, $R^4$ et $R^5$ étant choisis à chaque occurrence de manière identique ou différente dans le groupe constitué par H, le deutérium, $N(R^6)_2$, OH, $Si(R^6)_3$, $B(OR^6)_2$, $OSO_2R^6$, $CF_3$, CN, F, Br, I, un groupe alkyle, alcoxy ou thioalcoxy linéaire de 1 à 40 atomes C, qui peut à chaque fois être substitué avec un ou plusieurs radicaux $R^6$, et un ou plusieurs groupes $CH_2$ non voisins pouvant être remplacés par $R^6C{=}CR^6$, C=C, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S ou $CONR^6$, et un ou plusieurs atomes H pouvant être remplacés par le deutérium, CN, $CF_3$ ou $NO_2$ ;
un groupe alcényle ou alcynyle linéaire de 2 à 40 atomes C, qui peut à chaque fois être substitué avec un ou plusieurs radicaux $R^6$ et un ou plusieurs groupes $CH_2$ non voisins pouvant être remplacés par $R^6C{=}CR^6$, C=C, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S ou $CONR^6$, et un ou plusieurs atomes H pouvant être remplacés par le deutérium, CN, $CF_3$ ou $NO_2$ ;
un groupe alkyle, alcényle, alcynyle, alcoxy ou thioalcoxy ramifié ou cyclique de 3 à 40 atomes C, qui peut à chaque fois être substitué avec un ou plusieurs radicaux $R^6$ et un ou plusieurs groupes $CH_2$ non voisins pouvant être remplacés par $R^6C{=}CR^6$, C=C, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S ou $CONR^6$, et un ou plusieurs atomes H pouvant être remplacés par le deutérium, CN, $CF_3$ ou $NO_2$ ;
un système cyclique aromatique de 5 à 60 atomes de cycle aromatiques, qui peut être substitué avec un ou plusieurs radicaux $R^6$,
un système cyclique hétéroaromatique de 5 à 60 atomes de cycle aromatiques, qui peut être substitué avec un ou plusieurs radicaux $R^6$,
un groupe aryloxy ou hétéroaryloxy de 5 à 60 atomes de cycle aromatiques, qui peut être substitué avec un ou plusieurs radicaux $R^6$, et
un groupe diarylamino, un groupe dihétéroarylamino ou un groupe arylhétéroarylamino de 10 à 40 atomes de cycle aromatiques, qui peut être substitué avec un ou plusieurs radicaux $R^6$ ;
les $R^6$ étant choisis à chaque occurrence de manière identique ou différente dans le groupe constitué par
H, le deutérium, $N(R^7)_2$, OH, $Si(R^7)_3$, $B(OR^7)_2$, $OSO_2R^7$, $CF_3$, CN, F, Br, I, un groupe alkyle, alcoxy ou thioalcoxy linéaire de 1 à 40 atomes C, qui peut à chaque fois être substitué avec un ou plusieurs radicaux $R^7$ et un ou plusieurs groupes $CH_2$ non voisins pouvant être remplacés par $R^7C{=}CR^7$, C≡C, $Si(R^7)_2$, $Ge(R^7)_2$, $Sn(R^7)_2$, C=O, C=S, C=Se, $C{=}NR^7$, $P({=}O)(R^7)$, SO, $SO_2$, $NR^7$, O, S ou $CONR^7$, et un ou plusieurs atomes H pouvant être remplacés par le deutérium, CN, $CF_3$ ou $NO_2$ ;
un groupe alcényle ou alcynyle linéaire de 2 à 40 atomes C, qui peut à chaque fois être substitué avec un ou plusieurs radicaux $R^7$ et un ou plusieurs groupes $CH_2$ non voisins pouvant être remplacés par $R^7C{=}CR^7$, C=C,

$Si(R^7)_2$, $Ge(R^7)_2$, $Sn(R^7)_2$, C=O, C=S, C=Se, C=$NR^7$, P(=O)($R^7$), SO, $SO_2$, $NR^7$, O, S ou $CONR^7$, et un ou plusieurs atomes H pouvant être remplacés par le deutérium, CN, $CF_3$ ou $NO_2$ ;
un groupe alkyle, alcényle, alcynyle, alcoxy ou thioalcoxy ramifié ou cyclique de 3 à 40 atomes C, qui peut à chaque fois être substitué avec un ou plusieurs radicaux $R^7$ et un ou plusieurs groupes $CH_2$ non voisins pouvant être remplacés par $R^7C=CR^7$, C=C, $Si(R^7)_2$, $Ge(R^7)_2$, $Sn(R^7)_2$, C=O, C=S, C=Se, C=$NR^7$, P(=O)($R^7$), SO, $SO_2$, $NR^7$, O, S ou $CONR^7$, et un ou plusieurs atomes H pouvant être remplacés par le deutérium, CN, $CF_3$ ou $NO_2$ ;
un système cyclique aromatique de 5 à 60 atomes de cycle aromatiques, qui peut être substitué avec un ou plusieurs radicaux $R^7$,
un système cyclique hétéroaromatique de 5 à 60 atomes de cycle aromatiques, qui peut être substitué avec un ou plusieurs radicaux $R^7$,
un groupe aryloxy ou hétéroaryloxy de 5 à 60 atomes de cycle aromatiques, qui peut être substitué avec un ou plusieurs radicaux $R^7$, et
un groupe diarylamino, un groupe dihétéroarylamino ou un groupe arylhétéroarylamino de 10 à 40 atomes de cycle aromatiques, qui peut être substitué avec un ou plusieurs radicaux $R^7$ ;
les $R^7$ étant choisis à chaque occurrence de manière identique ou différente dans le groupe constitué par
H, le deutérium, OH, $CF_3$, CN, F,
un groupe alkyle, alcoxy ou thioalcoxy linéaire de 1 à 5 atomes C, un groupe alcényle ou alcynyle linéaire de 2 à 5 atomes C, un groupe alkyle, alcényle, alcynyle, alcoxy ou thioalcoxy ramifié ou cyclique de 3 à 5 atomes C, un ou plusieurs atomes H pouvant à chaque fois être remplacés par le deutérium, CN, $CF_3$ ou $NO_2$ dans les groupes susmentionnés ;
un système cyclique aromatique ou hétéroaromatique de 5 à 60 atomes de cycle aromatiques, un groupe aryloxy ou hétéroaryloxy de 5 à 60 atomes de cycle aromatiques ; et un groupe diarylamino, un groupe dihétéroarylamino ou un groupe arylhétéroarylamino de 10 à 40 atomes de cycle aromatiques ; les radicaux $R^a$, $R^4$, $R^5$ ou $R^6$ pouvant également former avec un ou plusieurs autres radicaux $R^a$, $R^4$, $R^5$ ou $R^6$ un système cyclique mono- ou polycyclique, aliphatique, aromatique et/ou benzoannelé ;
et
exactement un radical choisi dans le groupe constitué par X et Y représentant le point de liaison de la simple liaison entre la première unité chimique et la deuxième unité chimique D.

2. Molécule organique selon la revendication 1, dans laquelle $R^1$, $R^2$ et $R^3$ représentent à chaque occurrence de manière identique ou différente H, méthyle ou phényle.

3. Molécule organique selon la revendication 1 ou 2, dans laquelle W représente CN.

4. Molécule organique selon les revendications 1 à 3, dans laquelle la deuxième unité chimique D est constituée d'une structure de formule IIb :

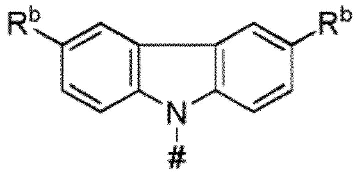

Formule IIb

dans laquelle

les $R^b$ sont choisis à chaque occurrence de manière identique ou différente dans le groupe constitué par
$N(R^6)_2$, OH, $Si(R^6)_3$, $B(OR^6)_2$, $OSO_2R^6$, $CF_3$, CN, F, Br, I,
un groupe alkyle, alcoxy ou thioalcoxy linéaire de 1 à 40 atomes C, qui peut à chaque fois être substitué avec un ou plusieurs radicaux $R^6$, et un ou plusieurs groupes $CH_2$ non voisins pouvant être remplacés par $R^6C=CR^6$, C=C, $Si(R^6)_2$, $Ge(R^6)_2$, $Sn(R^6)_2$, C=O, C=S, C=Se, C=$NR^6$, P(=O)($R^6$), SO, $SO_2$, $NR^6$, O, S ou $CONR^6$, et un ou plusieurs atomes H pouvant être remplacés par le deutérium, CN, $CF_3$ ou $NO_2$ ;
un groupe alcényle ou alcynyle linéaire de 2 à 40 atomes C, qui peut à chaque fois être substitué avec un ou plusieurs radicaux $R^6$, et un ou plusieurs groupes $CH_2$ non voisins pouvant être remplacés par $R^6C=CR^6$, C=C, $Si(R^6)_2$, $Ge(R^6)_2$, $Sn(R^6)_2$, C=O, C=S, C=Se, C=$NR^6$, P(=O)($R^6$), SO, $SO_2$, $NR^6$, O, S ou $CONR^6$, et un ou

plusieurs atomes H pouvant être remplacés par le deutérium, CN, $CF_3$ ou $NO_2$ ;
un groupe alkyle, alcényle, alcynyle, alcoxy ou thioalcoxy ramifié ou cyclique de 3 à 40 atomes C, qui peut à chaque fois être substitué avec un ou plusieurs radicaux $R^6$, et un ou plusieurs groupes $CH_2$ non voisins pouvant être remplacés par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$, et un ou plusieurs atomes H pouvant être remplacés par le deutérium, CN, $CF_3$ ou $NO_2$ ;
un système cyclique aromatique de 5 à 60 atomes de cycle aromatiques, qui peut être substitué avec un ou plusieurs radicaux $R^6$,
un système cyclique hétéroaromatique de 5 à 60 atomes de cycle aromatiques, qui peut être substitué avec un ou plusieurs radicaux $R^6$, un groupe aryloxy ou hétéroaryloxy de 5 à 60 atomes de cycle aromatiques, qui peut être substitué avec un ou plusieurs radicaux $R^6$, et
un groupe diarylamino, un groupe dihétéroarylamino ou un groupe arylhétéroarylamino de 10 à 40 atomes de cycle aromatiques, qui peut être substitué avec un ou plusieurs radicaux $R^6$ ;
et # et $R^6$ ayant les définitions indiquées dans la revendication 1.

**5.** Molécule organique selon les revendications 1 à 3, dans laquelle la deuxième unité chimique D est constituée par une structure de formule IIc :

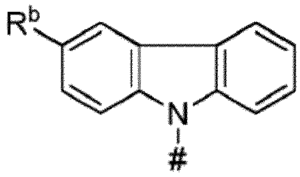

Formule IIc

dans laquelle

les $R^b$ sont choisis à chaque occurrence de manière identique ou différente dans le groupe constitué par $N(R^6)_2$, OH, $Si(R^6)_3$, $B(OR^6)_2$, $OSO_2R^6$, $CF_3$, CN, F, Br, I,
un groupe alkyle, alcoxy ou thioalcoxy linéaire de 1 à 40 atomes C, qui peut à chaque fois être substitué avec un ou plusieurs radicaux $R^6$,
et un ou plusieurs groupes $CH_2$ non voisins pouvant être remplacés par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$, et un ou plusieurs atomes H pouvant être remplacés par le deutérium, CN, $CF_3$ ou $NO_2$ ;
un groupe alcényle ou alcynyle linéaire de 2 à 40 atomes C, qui peut à chaque fois être substitué avec un ou plusieurs radicaux $R^6$, et un ou plusieurs groupes $CH_2$ non voisins pouvant être remplacés par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$, et un ou plusieurs atomes H pouvant être remplacés par le deutérium, CN, $CF_3$ ou $NO_2$ ;
un groupe alkyle, alcényle, alcynyle, alcoxy ou thioalcoxy ramifié ou cyclique de 3 à 40 atomes C, qui peut à chaque fois être substitué avec un ou plusieurs radicaux $R^6$, et un ou plusieurs groupes $CH_2$ non voisins pouvant être remplacés par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$, et un ou plusieurs atomes H pouvant être remplacés par le deutérium, CN, $CF_3$ ou $NO_2$ ;
un système cyclique aromatique de 5 à 60 atomes de cycle aromatiques, qui peut être substitué avec un ou plusieurs radicaux $R^6$,
un système cyclique hétéroaromatique de 5 à 60 atomes de cycle aromatiques, qui peut être substitué avec un ou plusieurs radicaux $R^6$, un groupe aryloxy ou hétéroaryloxy de 5 à 60 atomes de cycle aromatiques, qui peut être substitué avec un ou plusieurs radicaux $R^6$, et
un groupe diarylamino, un groupe dihétéroarylamino ou un groupe arylhétéroarylamino de 10 à 40 atomes de cycle aromatiques, qui peut être substitué avec un ou plusieurs radicaux $R^6$ ;
les définitions indiquées dans la revendication 1 s'appliquant pour le reste.

**6.** Molécule organique selon la revendication 4 ou 5, dans laquelle les $R^b$ représentent à chaque occurrence de manière identique ou différente : Me, $^i$Pr, $^t$Bu, CN, $CF_3$, ou Ph, qui peut à chaque fois être substitué avec un ou plusieurs radicaux choisis parmi Me, $^i$Pr, $^t$Bu, CN, $CF_3$ et Ph, pyridinyle, qui peut à chaque fois être substitué avec un ou plusieurs radicaux choisis parmi Me, $^i$Pr, $^t$Bu, CN, $CF_3$ et Ph, pyrimidinyle, qui peut à chaque fois être substitué avec un ou plusieurs radicaux choisis parmi Me, $^i$Pr, $^t$Bu, CN, $CF_3$ et Ph, triazinyle, qui peut à chaque fois être substitué avec un ou plusieurs radicaux choisis parmi Me, $^i$Pr, $^t$Bu, CN, $CF_3$ et Ph, carbazolyle qui peut à chaque fois être

substitué avec un ou plusieurs radicaux choisis parmi Me, $^{i}P_{r}$, $^{t}B_{u}$, CN, $CF_3$ et Ph, ou $N(Ph)_2$.

**7.** Procédé de fabrication d'une molécule organique selon les revendications 1 à 6, selon lequel un acide 4-pyridineboronique substitué par $R^1$ en position 3, 5 et substitué par $R^3$ en position 2,6 ou un ester d'acide 4-pyridineboronique correspondant substitué par $R^1$ en position 3, 5 et substitué par $R^3$ en position 2, 6 est utilisé en tant que réactif.

**8.** Utilisation d'une molécule organique selon les revendications 1 à 6 en tant qu'émetteur luminescent et/ou en tant que matériau hôte et/ou en tant que matériau de transport d'électrons et/ou en tant que matériau d'injection de trous et/ou en tant que matériau de blocage de trous dans un dispositif optoélectronique organique.

**9.** Utilisation selon la revendication 8, dans laquelle le dispositif optoélectronique organique est choisi dans le groupe constitué par :

- les diodes organiques électroluminescentes (OLED),
- les cellules électrochimiques électroluminescentes,
- les capteurs à OLED, notamment dans des capteurs de gaz et de vapeur non blindés hermétiquement vers l'extérieur,
- les diodes organiques,
- les cellules solaires organiques,
- les transistors organiques,
- les transistors à effet de champ organiques,
- les lasers organiques et
- les éléments de conversion descendante.

**10.** Composition, comprenant ou constituée par :

(a) au moins une molécule organique selon l'une quelconque des revendications 1 à 6, notamment sous la forme d'un émetteur et/ou d'un hôte, et
(b) un ou plusieurs matériaux émetteurs et/ou hôtes, qui sont différents de la molécule selon les revendications 1 à 6 ; et
(c) éventuellement un ou plusieurs colorants et/ou un ou plusieurs solvants.

**11.** Dispositif optoélectronique organique, comprenant une molécule organique selon les revendications 1 à 6 ou une composition selon la revendication 10, notamment configurée sous la forme d'un dispositif choisi dans le groupe constitué par une diode organique électroluminescente (OLED), une cellule électrochimique électroluminescente, un capteur d'OLED, notamment des capteurs de gaz et de vapeur non blindés hermétiquement vers l'extérieur, une diode organique, une cellule solaire organique, un transistor organique, un transistor à effet de champ organique, un laser organique et un élément de conversion descendante.

**12.** Dispositif optoélectronique organique selon la revendication 11, comprenant :

- un substrat,
- une anode et
- une cathode, l'anode ou la cathode étant appliquée sur le substrat et
- au moins une couche électroluminescente, qui est agencée entre l'anode et la cathode, et comprend une molécule organique selon les revendications 1 à 6 ou une composition selon la revendication 10.

**13.** Procédé de fabrication d'un composant optoélectronique, selon lequel une molécule organique selon les revendications 1 à 6 est utilisée, notamment comprenant le traitement de la molécule organique par un procédé d'évaporation sous vide ou à partir d'une solution.

Figur 1

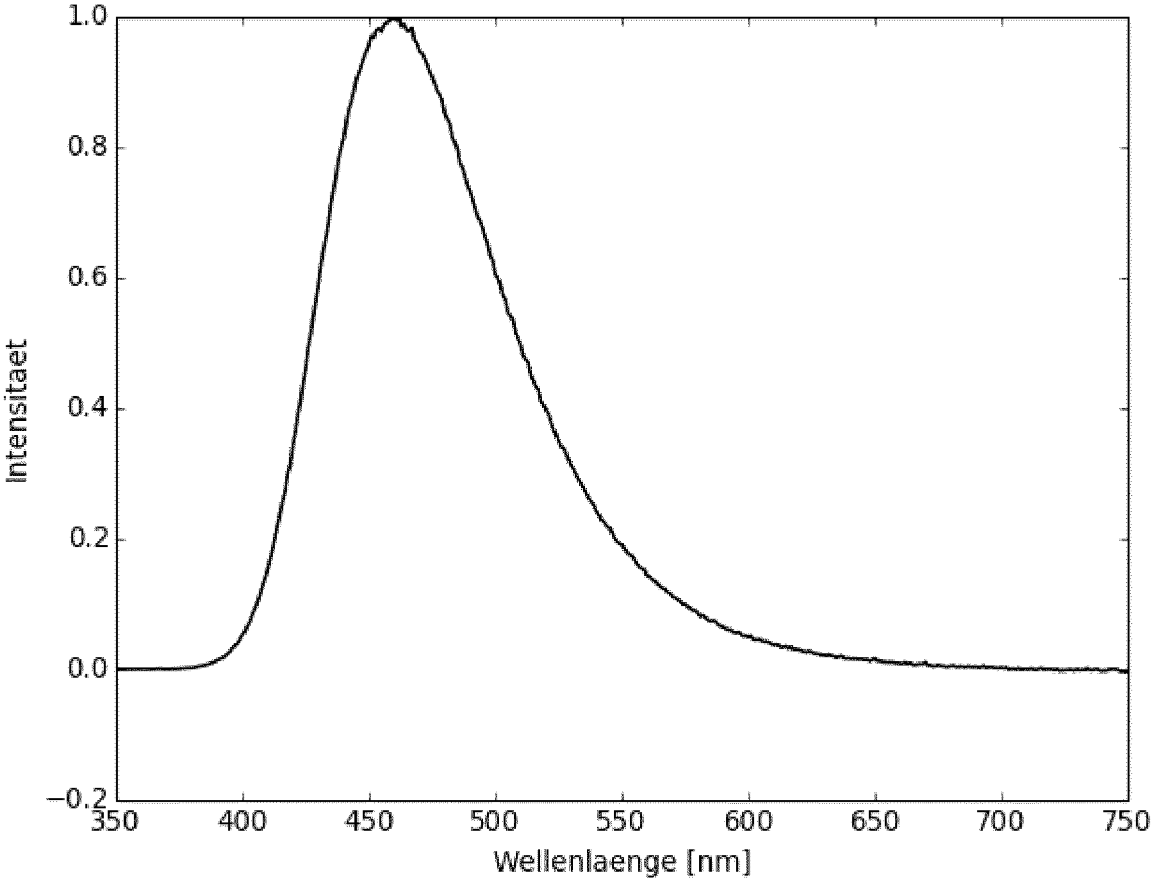
1.0
0.8
0.6
0.4
0.2
0.0
−0.2
Intensitaet
350
400
450
500
550
600
650
700
750
Wellenlaenge [nm]

Figur 2

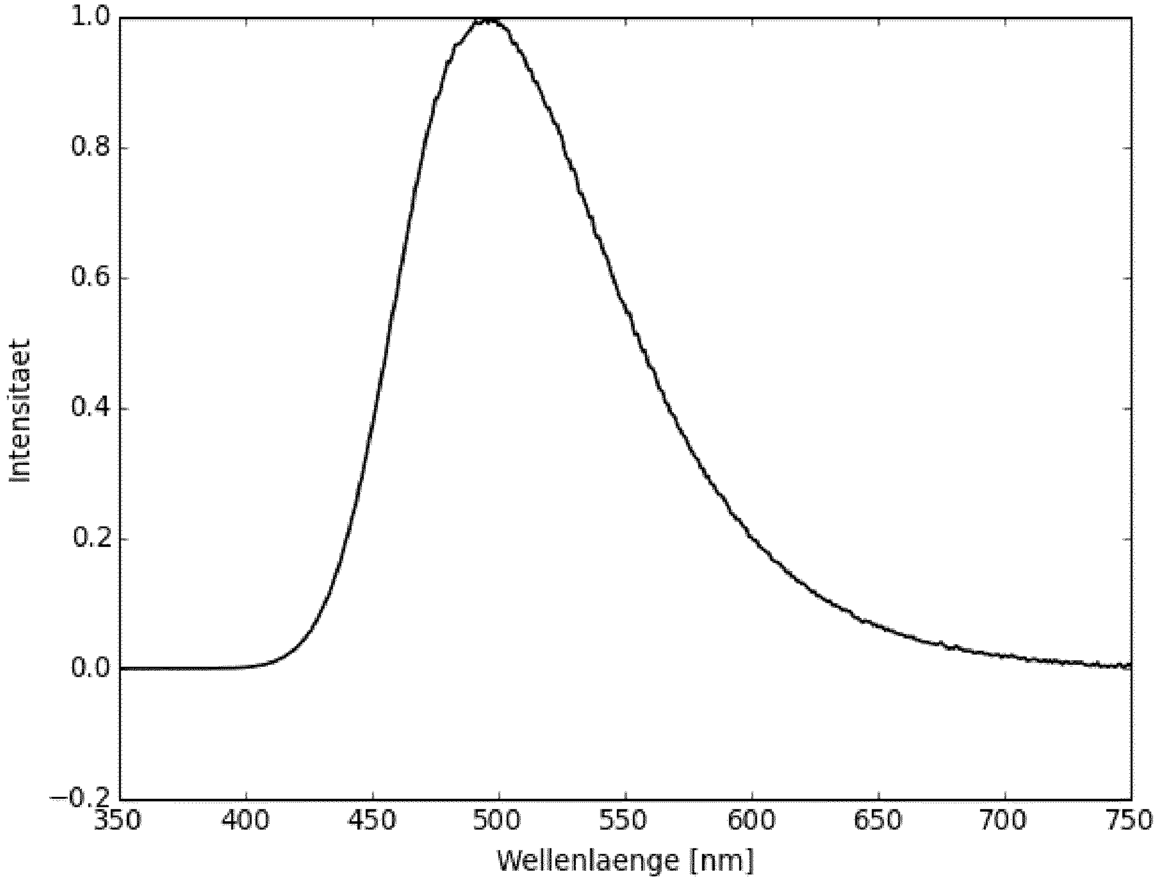
1.0
0.8
0.6
0.4
0.2
0.0
−0.2
Intensitaet
350
400
450
500
550
600
650
700
750
Wellenlaenge [nm]

**Figur 3**

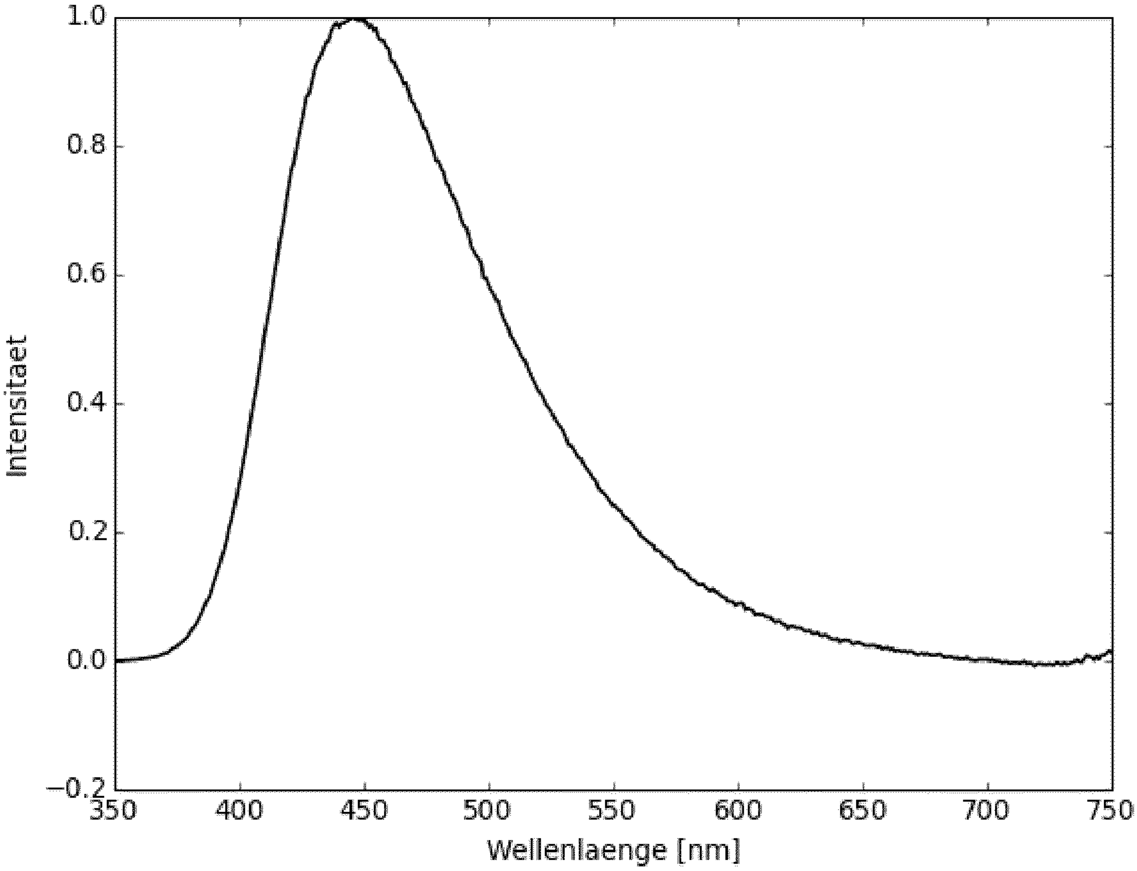
1.0
0.8
0.6
0.4
0.2
0.0
−0.2
Intensitaet
350
400
450
500
550
600
650
700
750
Wellenlaenge [nm]

**Figur 4**

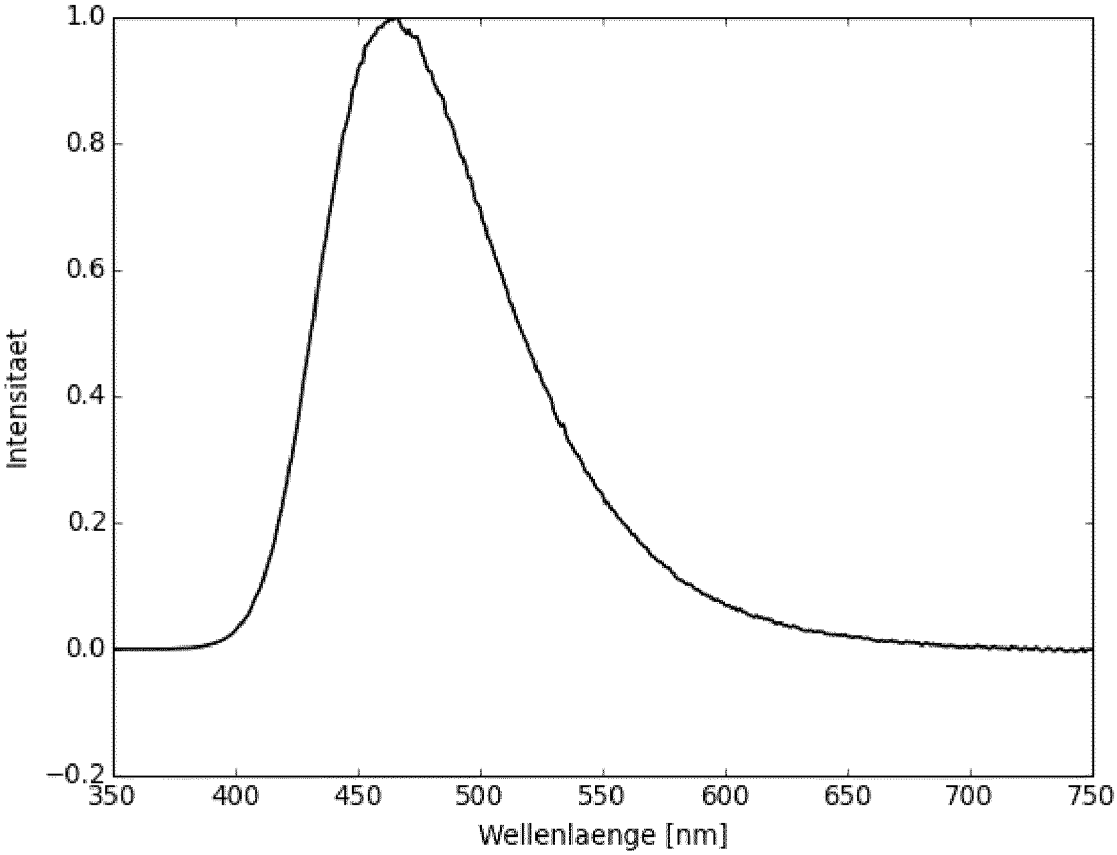
1.0
0.8
0.6
0.4
0.2
0.0
−0.2
Intensitaet
350
400
450
500
550
600
650
700
750
Wellenlaenge [nm]

**Figur 5**

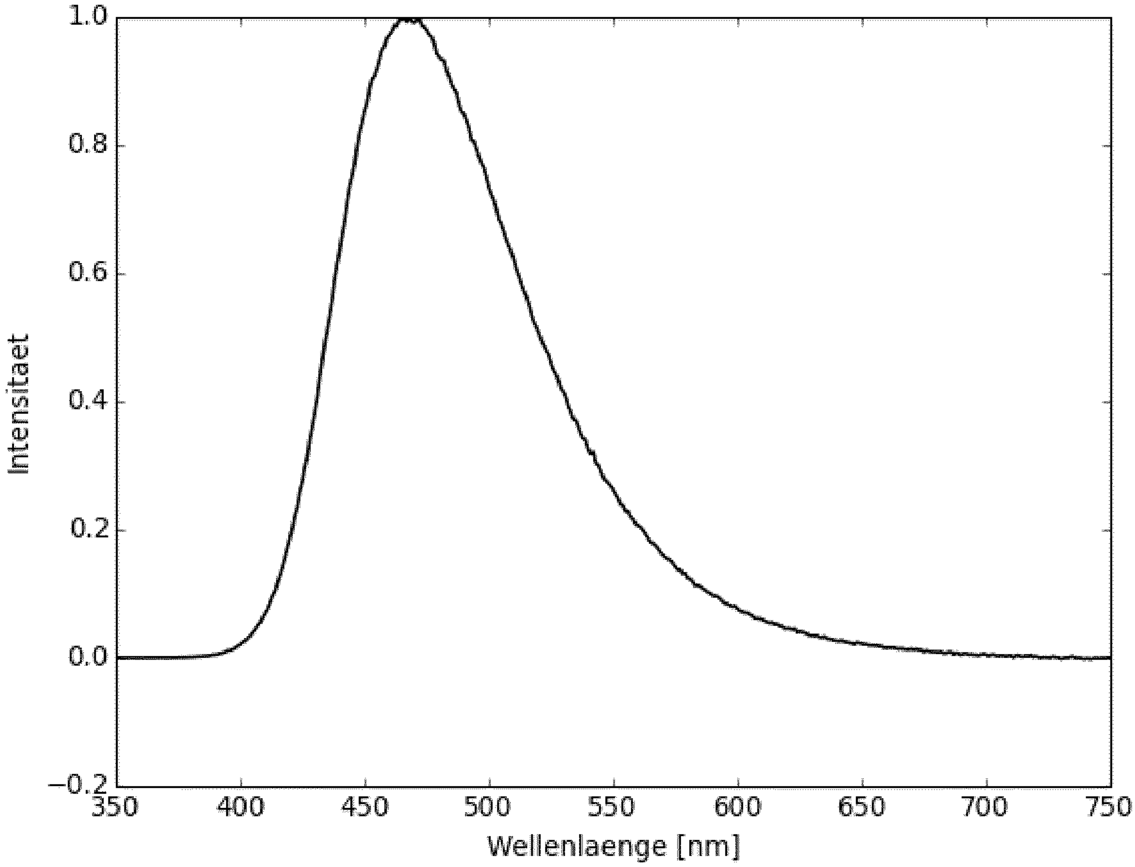
1.0
0.8
0.6
0.4
0.2
0.0
−0.2
Intensitaet
350
400
450
500
550
600
650
700
750
Wellenlaenge [nm]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- KR 20140139307 A
- WO 2014146752 A1
- WO 2016024675 A1
- WO 2013100540 A1

### In der Beschreibung aufgeführte Nicht-Patentliteratur

- **M.E. THOMPSON et al.** *Chem. Mater.,* 2004, vol. 16, 4743